(19) Europäisches Patentamt
European Patent Office
Office européen des brevets

(11) EP 0 584 694 B1

(12) **EUROPÄISCHE PATENTSCHRIFT**

(45) Veröffentlichungstag und Bekanntmachung des Hinweises auf die Patenterteilung:
**21.01.1998 Patentblatt 1998/04**

(51) Int Cl.[6]: **C07K 5/02**, C07K 5/10, A61K 38/07

(21) Anmeldenummer: **93113143.7**

(22) Anmeldetag: **17.08.1993**

# (54) Imidazolidin-Derivate

Imidazolidin derivatives

Dérivés d'imidazolidine

(84) Benannte Vertragsstaaten:
**AT BE CH DE DK ES FR GB IT LI NL SE**

(30) Priorität: **28.08.1992 DE 4228717**

(43) Veröffentlichungstag der Anmeldung:
**02.03.1994 Patentblatt 1994/09**

(73) Patentinhaber: **HOECHST AKTIENGESELLSCHAFT**
**65926 Frankfurt am Main (DE)**

(72) Erfinder:
- **Zoller, Gerhard, Dr.**
  **D-61137 Schöneck (DE)**
- **Jablonka, Bernd, Dr.**
  **D-65812 Bad Soden (DE)**
- **Just, Melitta, Dr.**
  **D-63225 Langen (DE)**
- **Klingler, Otmar, Dr.**
  **D-63110 Rodgau (DE)**
- **Breipohl, Gerhard, Dr.**
  **D-60529 Frankfurt am Main (DE)**
- **Knolle, Jochen, Dr.**
  **D-65830 Kriftel (DE)**
- **König, Wolfgang, Dr.**
  **D-94375 Stallwang (DE)**

(56) Entgegenhaltungen:
**EP-A- 0 449 079**

Bemerkungen:
Die Akte enthält technische Angaben, die nach dem Eingang der Anmeldung eingereicht wurden und die nicht in dieser Patentschrift enthalten sind.

EP 0 584 694 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft Imidazolidin-Derivate, ihre Herstellung und ihre Verwendung als Hemmstoffe der Blutplättchenaggregation.

In der EP-A 449 079 sind Hydantoinderivate mit thrombozytenaggregationshemmender Wirkung beschrieben. Weitere Forschungsarbeiten haben gezeigt, daß auch die Verbindungen der vorliegenden Erfindung starke Hemmstoffe der Blutplättchenaggregation sind.

Die vorliegende Erfindung betrifft Verbindungen der allgemeinen Formel I

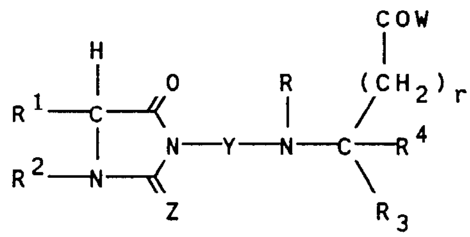

(I)

worin

**Y** $-(CH_2)_m-CO-$, wobei m für eine ganze Zahl von 1 bis 4 steht, oder

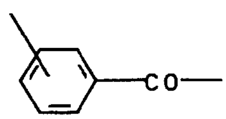

bedeutet;

**r** eine Zahl von 0 bis 3 bedeutet;

**Z** Sauerstoff oder Schwefel bedeutet;

**W** $(C_1-C_{28})$-Alkoxy, $(C_6-C_{14})$-Aryl-$(C_1-C_8)$-alkoxy, $(C_6-C_{14})$-Aryloxy, Amino oder Mono- oder Di-$(C_1-C_{18})$-alkylamino bedeutet;

**R** Wasserstoff oder $(C_1-C_6)$-Alkyl bedeutet;

**$R^1$** $-(CH_2)_n-NH-X$, wobei n für eine ganze Zahl von 1 bis 6 steht, $-(CH_2)_p-C_6H_4-(CH_2)_q-NH-X$ oder $-(CH_2)_p-C_6H_4-(CH_2)_q-C(=NX^2)-NH_2$, wobei p für eine ganze Zahl von 1 bis 3 und q für eine Zahl von 0 bis 2 steht, bedeutet, wobei aber anstelle von
$\rangle CH-R^1$ auch $\rangle C=CH-C_6H_4-X^1$ stehen kann ;

**X** Wasserstoff, $(C_1-C_6)$-Alkyl, $(C_1-C_6)$-Alkoxycarbonyl, $(C_1-C_6)$-Alkylcarbonyl, $(C_1-C_{18})$-Alkylcarbonyloxy-$(C_1-C_6)$-alkoxycarbonyl, $(C_6-C_{14})$-Aryloxycarbonyl, $(C_6-C_{14})$-Aryl-$(C_1-C_6)$-alkoxycarbonyl, Cyano, Hydroxy, $(C_1-C_6)$-Alkoxy, Amino oder einen Rest der Formel II

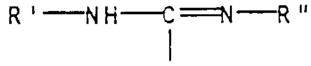

(II)

bedeutet, worin R' und R" unabhängig voneinander für Wasserstoff, $(C_1-C_6)$-Alkyl, $(C_1-C_6)$-Alkoxycarbonyl, $(C_1-$

$C_6$)-Alkylcarbonyl, ($C_1$-$C_{18}$)-Alkylcarbonyloxy-($C_1$-$C_6$)-alkoxycarbonyl, ($C_6$-$C_{14}$)-Aryloxycarbonyl, ($C_6$-$C_{14}$)-Aryl-($C_1$-$C_6$)-alkoxycarbonyl, Cyano, Hydroxy, ($C_1$-$C_6$)-Alkoxy oder Amino stehen;

**$X^1$** -$(CH_2)_q$-NH-X oder -$(CH_2)_q$-C(=$NX^2$)-$NH_2$ bedeutet;

**$X^2$** Wasserstoff, ($C_1$-$C_6$)-Alkyl, ($C_1$-$C_6$)-Alkoxycarbonyl, ($C_1$-$C_6$)-Alkylcarbonyl, ($C_1$-$C_{18}$)-Alkylcarbonyloxy-($C_1$-$C_6$)-alkoxycarbonyl, ($C_6$-$C_{14}$)-Aryloxycarbonyl, ($C_6$-$C_{14}$)-Aryl-($C_1$-$C_6$)-alkoxycarbonyl, Cyano, Hydroxy, ($C_1$-$C_6$)-Alkoxy oder Amino bedeutet;

**$R^2$** Wasserstoff oder ($C_1$-$C_6$)-Alkyl bedeutet;

**$R^3$** Wasserstoff oder Phenyl bedeutet;

**$R^4$** Wasserstoff, -$COOR^5$, -CO-N($CH_3$)-$R^5$ oder -CO-NH-$R^5$ bedeutet;

**$R^5$** Wasserstoff oder ($C_1$-$C_{28}$)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-($C_1$-$C_{18}$)-Alkylaminocarbonyl, Amino-($C_2$-$C_{14}$)-alkylaminocarbonyl, Amino-($C_1$-$C_3$)alkylphenyl-($C_1$-$C_3$)alkylaminocarbonyl, ($C_1$-$C_{18}$)-Alkylcarbonylamino-($C_1$-$C_3$)-alkylphenyl-($C_1$-$C_3$)alkylaminocarbonyl, ($C_1$-$C_{18}$)-Alkylcarbonylamino-($C_2$-$C_{14}$)-alkylaminocarbonyl, Phenyl-($C_1$-$C_8$)-alkoxycarbonyl, Amino, Mercapto, ($C_1$-$C_{18}$)-Alkoxy, ($C_1$-$C_{18}$)-Alkoxycarbonyl, ($C_3$-$C_8$)-Cycloalkyl, Halogen, Nitro, Trifluormethyl oder einen Rest $R^6$ substituiert ist;

**$R^6$** ($C_6$-$C_{14}$)-Aryl, ($C_6$-$C_{14}$)-Aryl-($C_1$-$C_8$)-Alkyl, einen mono- oder bicyclischen 5- bis 12-gliedrigen heterocyclischen Ring, der aromatisch, teilhydriert oder vollständig hydriert sein kann und der als Heteroelement ein, zwei oder drei gleiche oder verschiedene Stickstoff-, Sauerstoff- oder Schwefel-Atome enthalten-kann, oder einen Rest $R^7$ bedeutet, wobei der Aryl- und unabhängig davon der Heterocyclus-Rest gegebenenfalls ein- oder mehrfach durch gleiche oder verschiedene Reste aus der Reihe ($C_1$-$C_{18}$)-Alkyl, ($C_1$-$C_{18}$)-Alkoxy, Halogen, Nitro oder Trifluormethyl substituiert sein können;

**$R^7$** -$NR^8R^9$, -$OR^8$, -$SR^8$, eine Aminosäureseitenkette, einen natürlichen oder unnatürlichen Aminosäure-, Iminosäure- oder Dipeptid-Rest, sowie deren Ester und Amide, wobei freie funktionelle Gruppen durch in der Peptidchemie übliche Schutzgruppen geschützt sein können, oder einen Rest -$COR^{7'}$, worin $R^{7'}$ wie $R^7$ definiert ist, bedeutet;

**$R^8$** Wasserstoff, ($C_2$-$C_{18}$)-Alkyl, ($C_6$-$C_{14}$)-Aryl-($C_1$-$C_8$)-alkyl, ($C_1$-$C_{18}$)-Alkylcarbonyl, ($C_1$-$C_{18}$)-Alkoxycarbonyl, ($C_6$-$C_{14}$)-Arylcarbonyl, ($C_6$-$C_{14}$)-Aryl-($C_1$-$C_8$)-alkylcarbonyl, ($C_6$-$C_{14}$)-Aryl-($C_1$-$C_{18}$)-alkoxycarbonyl, wobei die Alkylgruppen gegebenenfalls durch eine Aminogruppe substituiert sein können, einen natürlichen oder unnatürlichen Aminosäure-, Iminosäure- oder einen Dipeptid-Rest bedeutet; und

**$R^9$** Wasserstoff, ($C_1$-$C_{18}$)-Alkyl, ($C_6$-$C_{14}$)-Aryl oder ($C_6$-$C_{14}$)-Aryl-($C_1$-$C_8$)-alkyl bedeutet;

sowie deren physiologisch verträgliche Salze,
wobei Verbindungen der allgemeinen Formel I ausgeschlossen sind, in denen gleichzeitig

Y für -$CH_2$-CO- steht;
r die Zahl 1 bedeutet;
Z Sauerstoff bedeutet;
W tert.-Butoxy bedeutet;
R, $R^2$ und $R^3$ Wasserstoff bedeuten;
$R^1$ -$(CH_2)_k$-$NHX^a$ bedeutet, wobei k für 3 oder 4 steht;
$X^a$ Wasserstoff, ($C_1$-$C_6$)-Alkyl oder einen Rest der Formel

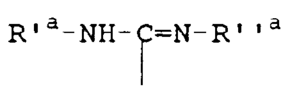

bedeutet, in der $R'^a$ und $R''^a$ unabhängig voneinander Wasserstoff oder ($C_1$-$C_6$)-Alkyl bedeuten;
$R^4$ -$CONHR^5$ bedeutet.

Alkylreste können geradkettig oder verzweigt sein. Bevorzugte Alkylreste sind Methyl, Ethyl, n-Propyl, i-Propyl, n-Butyl, i-Butyl, sek-Butyl und tert.-Butyl. Entsprechendes gilt für Reste wie Alkoxy, Alkoxycarbonyl oder Aralkyl.

($C_3$-$C_8$)-Cycloalkylreste sind insbesondere Cyclopropyl, Cyclobutyl, Cyclopentyl, Cyclohexyl, Cycloheptyl und Cyclooctyl, die aber auch durch beispielsweise ($C_1$-$C_4$)-Alkyl substituiert sein können. Beispiele für substituierte Cycloalkylreste sind 4-Methylcyclohexyl und 2,3-Dimethylcyclopentyl.

($C_6$-$C_{14}$)-Arylgruppen sind beispielsweise Phenyl, Naphthyl, Biphenylyl oder Fluorenyl, wobei Phenyl und Naphthyl bevorzugt sind. Entsprechendes gilt für Reste wie Aralkyl oder Arylcarbonyl. Aralkylreste sind insbesondere Benzyl sowie 1- und 2-Naphthylmethyl, die auch substituiert sein können. Substituierte Aralkylreste sind beispielsweise Halobenzyl oder ($C_1$-$C_4$)-Alkoxybenzyl.

Ist Phenyl zweifach substituiert, können die Substituenten in 1,2-, 1,3- oder 1,4-Position zueinander stehen. Die 1,3- sowie die 1,4-Position sind bevorzugt.

Heterocyclen im Sinne vorstehender Definitionen sind beispielsweise Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindazolyl, Indazolyl, Phthalazinyl, Chinolyl, Isochinolyl, Chinoxalinyl, Chinazolinyl, Cinnolinyl oder ein benzanelliertes, cyclopenta-, cyclohexa- oder cyclohepta-anelliertes Derivat dieser Reste.

Diese Heterocyclen können an einem Stickstoffatom durch Oxide, ($C_1$-$C_7$)-Alkyl, z. B. Methyl oder Ethyl, Phenyl oder Phenyl-($C_1$-$C_4$)-alkyl, z. B. Benzyl und/oder an einem oder mehreren Kohlenstoffatomen durch ($C_1$-$C_4$)-Alkyl, Halogen, Hydroxy, ($C_1$-$C_4$)-Alkoxy, z. B. Methoxy, Phenyl-($C_1$-$C_4$)-alkoxy, z. B. Benzyloxy oder Oxo substituiert und teilweise oder vollständig gesättigt sein.

Derartige Reste sind beispielsweise 2- oder 3-Pyrrolyl, Phenyl-pyrrolyl, z. B. 4- oder 5-Phenyl-2-pyrrolyl, 2-Furyl, 2-Thienyl, 4-Imidazolyl, Methyl-imidazolyl, z. B. 1-Methyl-2-, 4- oder 5-imidazolyl, 1,3-Thiazol-2-yl, 2-, 3- oder 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, z. B. 1-Methyl-, 5-Methyl-, 5-Methoxy, 5-Benzyloxy, 5-Chlor oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3-oder 4-Chinolyl, 1-, 3- oder 4-Isochinolyl, 1-Oxo-1,2-dihydro-3-isochinolyl, 2-Chinoxalinyl, 2-Benzofuranyl, 2-Benzothienyl, 2-Benzoxazolyl oder Benzothiazolyl. Teilhydrierte oder vollständig hydrierte heterocyclische Ringe sind beispielsweise Dihydropyridinyl, Pyrrolidinyl, z. B. 2-, 3- oder 4-N-methylpyrrolidinyl, Piperazinyl, Morpholinyl, Thiomorpholinyl, Tetrahydrothienyl, Benzodioxolanyl.

Halogen steht für Fluor, Chlor, Brom oder Jod, insbesondere für Fluor oder Chlor.

Natürliche und unnatürliche Aminosäuren können, falls chiral, in der D- oder L-Form vorliegen. Bevorzugt sind α-Aminosäuren. Beispielsweise seien genannt (vgl. Houben-Weyl, Methoden der organischen Chemie, Band XV/1 und 2, Stuttgart, 1974):

Aad, Abu γAbu, ABz, 2ABz, εAca,
Ach, Acp, Adpd, Ahb, Aib, βAib, Ala, βAla,
ΔAla, Alg, All, Ama, Amt, Ape,
Apm, Apr, Arg, Asn, Asp, Asu, Aze, Azi, Bai, Bph, Can,
Cit, Cys, $(Cys)_2$, Cyta, Daad, Dab, Dadd, Dap, Dapm, Dasu,
Djen, Dpa, Dtc, Fel, Gln, Glu, Gly, Guv, hAla, hArg,
hCys, hGln, hGlu, His, hIle, hLeu, hLys, hMet, hPhe,
hPro, hSer, hThr, hTrp, hTyr, Hyl, Hyp, 3Hyp,
Ile, Ise, Iva, Kyn, Lant, Lcn, Leu, Lsg, Lys, βLys,
ΔLys, Met, Mim, Min, nArg, Nle,
Nva, Oly, Orn, Pan, Pec, Pen, Phe, Phg, Pic, Pro,
ΔPro, Pse, Pya, Pyr, Pza, Qin,
Ros, Sar, Sec, Sem, Ser, Thi, βThi, Thr, Thy, Thx, Tia,
Tle, Tly, Trp, Trta, Tyr, Val, Tbg, Npg, Chg, Cha, Thia,
2,2-Diphenylaminoessigsäure, 2-(p-Tolyl)-2-phenylaminoessigsäure, 2-(p-Chlorphenyl)aminoessigsäure.

Unter Aminosäureseitenketten werden Seitenketten von natürlichen oder unnatürlichen Aminosäuren verstanden.

Als Rest einer Iminosäure kommen insbesondere Reste von Heterocyclen aus der folgenden Gruppe in Betracht: Pyrrolidin-2-carbonsäure; Piperidin-2-carbonsäure; Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Aza-bicyclo-[2.2.2]octan-3-carbonsäure; 2-Azabicyclo[2.2.1]heptan-3-carbonsäure; 2-Azabicyclo-[3.1.0]hexan-3-carbonsäure; 2-Azaspiro[4.4]nonan-3-carbonsäure; 2-Azaspiro[4.5]decan-3-carbonsäure; Spiro(bicyclo[2.2.1]-heptan)-2,3-pyrrolidin-5-carbonsäure; Spiro(bicyclo[2.2.2]octan)-2,3-pyrrolidin-5-carbonsäure; 2-Azatricyclo[$4.3.0.1^{6,9}$]decan-3-carbonsäure; Decahydrocyclohepta[b]pyrrol-2-carbonsäure; Decahydrocycloocta[c]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure; Hydroxyprolin-2-carbonsäure; die alle gegebenenfalls substituiert sein können (siehe folgende Formeln):

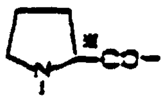 ; 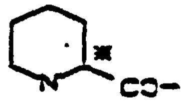 ;

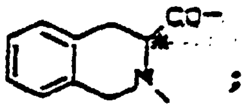 ; 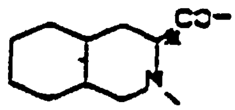 ; 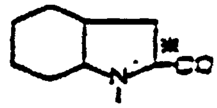 ; 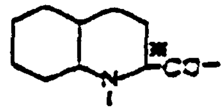 ;

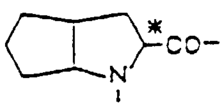 ; 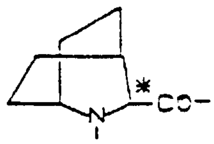 ; 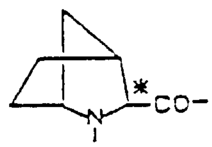 ; 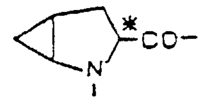 ;

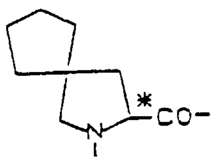 ; 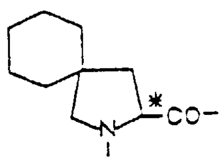 ; 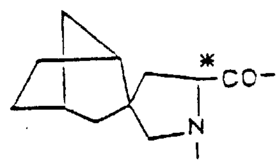 ;

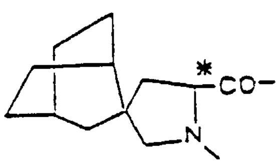 ; 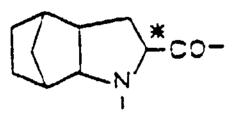 ; 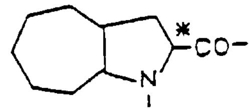 ;

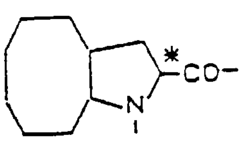 ; 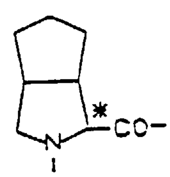 ; 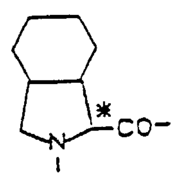 ;

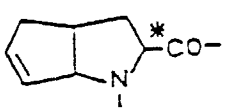 ; 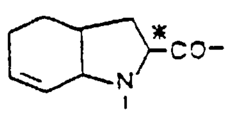 ; 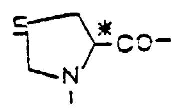 ;

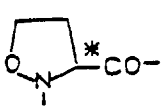 ; 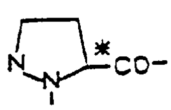 ; 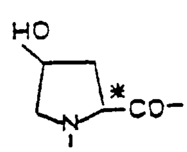 ;

Die oben genannten Resten zugrundeliegenden Heterocyclen sind beispielsweise bekannt aus
US-A 4,344,949; US-A 4,374,847; US-A 4,350,704;
EP-A 29,488; EP-A 31,741; EP-A 46,953; EP-A 49,605;
EP-A 49,658; EP-A 50,800; EP-A 51,020; EP-A 52,870;
EP-A 79,022; EP-A 84,164; EP-A 89,637; EP-A 90,341;
EP-A 90,362; EP-A 105,102; EP-A 109,020; EP-A 111,873;
EP-A 271,865 und EP-A 344,682.

Dipeptide könne als Bausteine natürliche oder unnatürliche Aminosäuren und Iminosäuren enthalten. Ferner können die natürlichen oder unnatürlichen Aminosäuren, Iminosäuren und Dipeptide auch als Ester bzw. Amide vorliegen, wie z. B. Methylester, Ethylamid, Semicarbazid oder ω-Amino-($C_4$-$C_8$)-alkylamid.

Funktionelle Gruppen der Aminosäuren, Iminosäuren und Dipeptide können geschützt vorliegen. Geeignete Schutzgruppen wie z. B. Urethanschutzgruppen, Carboxylschutzgruppen und Seitenkettenschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt: Aloc, Pyoc, Fmoc, Tcboc, Z, Boc, Ddz, Bpoc, Adoc, Msc, Moc, Z($NO_2$), Z($Hal_n$), Bobz, Iboc, Adpoc, Mboc, Acm, tert.-Butyl, OBzl, ONbzl, OMbzl, Bzl, Mob, Pic, Trt.

Physiologisch verträgliche Salze der Verbindungen der allgemeinen Formel I sind insbesondere pharmazeutisch verwendbare oder nicht-toxische Salze.

Solche Salze werden beispielsweise von Verbindungen der allgemeinen Formel I, welche saure Gruppen, z.B. Carboxy, enthalten, mit Alkali- oder Erdalkalimetallen gebildet, wie z.B. Na, K, Mg und Ca, sowie mit physiologisch verträglichen organischen Aminen, wie z. B. Triethylamin und Tris-(2-hydroxy-ethyl)-amin.

Verbindungen der allgemeinen Formel I, welche basische Gruppen, z.B. eine Aminogruppe oder eine Guanidinogruppe enthalten, bilden mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure Salze.

Die erfindungsgemäßen Verbindungen der allgemeinen Formel I können optisch aktive Kohlenstoffatome enthalten und somit in Form reiner Enantiomerer oder in Form von Enantiomerengemischen vorliegen. Sowohl reine Enantiomere als auch Enantiomerengemische sind Gegenstand der vorliegenden Erfindung.

Die erfindungsgemäßen Verbindungen der allgemeinen Formel I können darüber hinaus bewegliche Wasserstoffatome enthalten, also in verschiedenen tautomeren Formen vorliegen. Auch diese Tautomeren sind Gegenstand der vorliegenden Erfindung.

Bevorzugte Verbindungen der allgemeinen Formel I sind solche, worin

**Y** -$(CH_2)_m$-CO-, wobei m für 1 oder 2 steht, oder

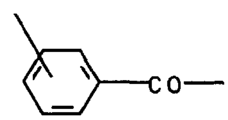

bedeutet;

**r** 1 bedeutet;

**Z** Sauerstoff oder Schwefel bedeutet;

**W** ($C_1$-$C_4$)-Alkoxy, insbesondere Methoxy, Ethoxy oder 2-Propyloxy, bedeutet;

**R** Wasserstoff bedeutet;

**R¹** -$(CH_2)_n$-NH-X, wobei n für eine ganze Zahl von 1 bis 4 steht, -$CH_2$-$C_6H_4$-$(CH_2)_q$-NH-X oder -$CH_2$-$C_6H_4$-$(CH_2)_q$-C-(=$NX^2$)-$NH_2$, wobei q für 0 oder 1 steht, bedeutet, wobei aber anstelle von
>CH-$R^1$ auch >C=CH-$C_6H_4$-$X^1$ stehen kann ;

**X** Wasserstoff, ($C_1$-$C_6$)-Alkoxycarbonyl, ($C_1$-$C_6$)-Alkylcarbonyl, ($C_6$-$C_{14}$)-Aryl-($C_1$-$C_6$)-alkoxycarbonyl oder einen Rest der Formel II

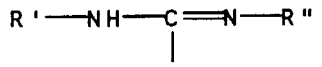 (II)

bedeutet, worin R' und R" unabhängig voneinander für Wasserstoff, $(C_1\text{-}C_6)$-Alkoxycarbonyl oder $(C_6\text{-}C_{14})$-Aryl $(C_1\text{-}C_6)$-alkoxycarbonyl stehen;

**$X^1$** $-(CH_2)_q$-NH-X oder $-C(=NX^2)-NH_2$, wobei q für 0 oder 1 steht, bedeutet;

**$X^2$** Wasserstoff, $(C_1\text{-}C_6)$-Alkoxycarbonyl, $(C_1\text{-}C_6)$-Alkylcarbonyl oder $(C_6\text{-}C_{14})$-Aryl-$(C_1\text{-}C_6)$-alkoxycarbonyl bedeutet;

**$R^2$** Wasserstoff oder Methyl bedeutet;

**$R^3$** Wasserstoff bedeutet; und

**$R^4$** -CO-NH-$R^5$ bedeutet, wobei -NH-$R^5$ für einen α-Aminosäurerest, dessen ω-Amino$(C_2\text{-}C_8)$-alkylamid oder dessen $(C_1\text{-}C_8)$-Alkyl- oder Benzylester steht.

Für -NH-$R^5$ stehende α-Aminosäurereste sind dabei besonders bevorzugt der Valin-, Lysin-, Phenylalanin-, Phenylglycin- oder der 4-Chlorphenylglycin-Rest. Ein besonders bevorzugtes ω-Amino-$(C_2\text{-}C_8)$-alkylamid ist das 4-Aminobutylamid.

Die erfindungsgemäßen Verbindungen der allgemeinen Formel I können hergestellt werden durch Fragmentkondensation einer Verbindung der allgemeinen Formel III

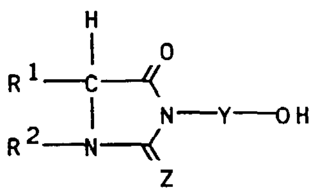 (III)

mit einer Verbindung der allgemeinen Formel IV

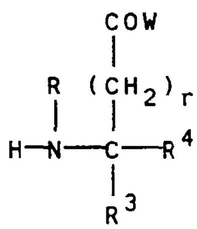 (IV)

wobei die Reste R, $R^1$ bis $R^4$ und r, Y, Z und W wie oben angegeben definiert sind.

Zur Kondensation der Verbindungen der allgemeinen Formel III mit denen der allgemeinen Formel IV verwendet man vorteilhafterweise die an sich bekannten Methoden der Peptidchemie (siehe z.B. Houben Weyl, Methoden der organischen Chemie, Band 15/1 und 15/2, Stuttgart, 1974).

Dazu ist es in der Regel nötig, daß in $R^1$ und $R^4$ und W enthaltene Aminogruppen durch reversible Schutzgruppen geschützt werden. Gleiches gilt für die Carboxylgruppen der Verbindung der allgemeinen Formel IV, die bevorzugt als Benzyl- oder tert.-Butylester vorliegen. Ein Aminogruppen-Schutz erübrigt sich, wenn die zu generierenden Aminogruppen als Nitro- oder Cyanogruppen vorliegen und erst nach der Kupplung durch Hydrierung gebildet werden.

Nach der Kupplung werden die vorhandenen Schutzgruppen in geeigneter Weise abgespalten. Beispielsweise können $NO_2$-Gruppen (Guanidinoschutz), Benzyloxycarbonylgruppen und Benzylester abhydriert werden. Schutzgruppen vom tert.-Butyltyp werden sauer gespalten, während der 9-Fluorenylmethyloxycarbonylrest durch sekundäre Amine entfernt wird.

Die Ausgangsverbindungen der allgemeinen Formel III können wie folgt erhalten werden:

Durch Umsetzung von Aminosäuren, N-Alkylaminosäuren oder bevorzugt deren Methyl-, Ethyl-, Benzyl- oder tert.-

Butylester, beispielsweise einer Verbindung der allgemeinen Formel V

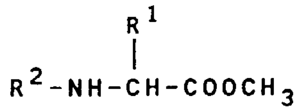

(V)

mit einem Isocyanatoalkancarbonsäureester, einem Isothiocyanatoalkancarbonsäureester oder einem Isocyanat oder Isothiocyanat der Aminobenzoesäure, beispielsweise der allgemeinen Formel VI

$$Z{=}C{=}N\text{-}Y\text{-}COOCH_3 \qquad \text{(VI)}$$

worin $R^1$, $R^2$, Y, Z und m wie oben angegeben definiert sind, erhält man Harnstoff- oder Thioharnstoffderivate, beispielsweise der allgemeinen Formel VII

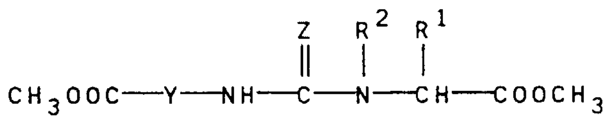

(VII)

die durch Erhitzen mit Säure unter Verseifung der Esterfunktionen zu Verbindungen der allgemeinen Formel III

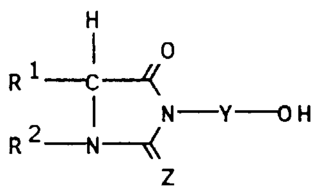

(III)

cyclisieren.

Während der Cyclisierung können Guanidinogruppen durch Schutzgruppen, wie $NO_2$ oder Mtr, blockiert werden. Ebenso müssen Aminogruppen in der Seitenkette in geschützter Form (beispielsweise als Boc- oder Z-Derivate) oder noch als $NO_2$- oder Cyanofunktion vorliegen, die später zur Aminogruppe reduziert oder im Falle der Cyanogruppe auch in die Formamidinogruppe umgewandelt werden kann.

Eine weitere Methode zur Herstellung von Verbindungen der allgemeinen Formel III ist die Umsetzung von Verbindungen der allgemeinen Formel XI

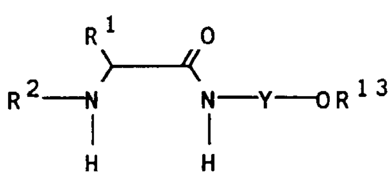

(XI)

worin $R^1$, $R^2$ und Y wie oben angegeben, definiert sind und $R^{13}$ beispielsweise ($C_1$-$C_6$)-Alkyl bedeutet, mit Phosgen, Thiophosgen oder entsprechenden Äquivalenten und anschließende Verseifung zu den entsprechenden Carbonsäuren (analog S. Goldschmidt und M. Wick, Liebigs Ann. Chem. 575 (1952) 217 - 231, C. Trapp, Chem. Ber. 61, (1928) 1431 - 1439).

Verbindungen der allgemeinen Formel IIIb

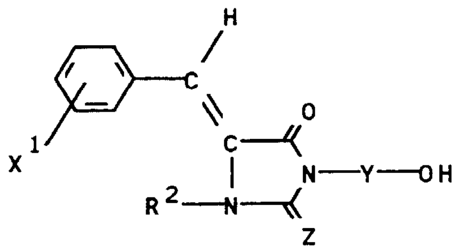

(IIIb)

können durch Umsetzung von Verbindungen der allgemeinen Formel VIII

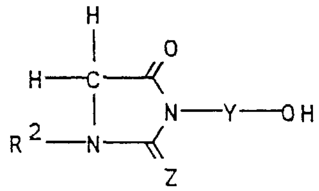

(VIII)

mit Aldehyden der allgemeinen Formel IX

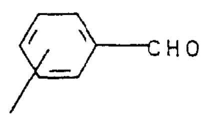

(IX)

analog Gränacher und Landolt, Helv. Chim. Acta 10 (1927) 808 erhalten werden.

Im übrigen entstehen Hydantoine der allgemeinen Formel Xa

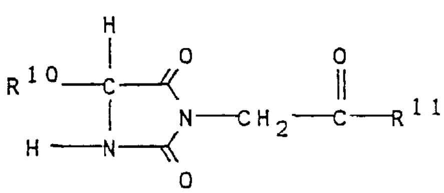

(Xa)

worin $R^{10}$ eine beliebige Aminosäureseitenkette und $R^{11}$ ein Amid, einen Aminosäure- oder einen Peptidrest bedeuten, ganz allgemein durch basische Behandlung von Alkyloxycarbonyl- oder Aralkyloxycarbonylpeptiden der allgemeinen Formel X

$$R^{12}\text{-O-CO-NH-CHR}^{10}\text{-CO-NH-CH}_2\text{-CO-R}^{11} \qquad (X)$$

worin $R^{10}$ und $R^{11}$ wie oben angegeben definiert sind und $R^{12}$ Benzyl oder tert.-Butyl bedeutet (J. S. Fruton und M. Bergmann, J. Biol. Chem. 145 (1942) 253 - 265; C. A. Dekker, S. P. Taylor, jr. und J. S. Fruton, J. Biol. Chem. 180 (1949) 155 - 173; M. E. Cox, H. G. Carg, J. Hollowood, J. M. Hugo, P. M. Scopes und G. T. Young, J. Chem. Soc. (1965) 6806 - 6813; W. Voelter und A. Altenburg, Liebigs Ann. Chem. (1983) 1641 - 1655; B. Schwenzer, E. Weber und G. Losse, J. Prakt. Chem. 327 (1985) 479 - 486). Dabei razemisiert jedoch die N-terminale Aminosäure und das Hydantoin hydrolysiert in das Harnstoffderivat

$$\text{HOCO-CHR}^{10}\text{-NH-CO-NH-CH}_2\text{-CO-R}^{11}$$

(W. Voelter und A. Altenburg, Liebigs Ann. Chem. (1983) 1641 - 1655).

Eine milde Methode ist dagegen die Zyklisierung zu den Hydantoinen aus Verbindungen der allgemeinen Formel X durch Behandlung mit Tetrabutylammoniumfluorid in Tetrahydrofuran unter Rückfluß (J. Pless, J. Org. Chem. 39 (1974) 2644 - 2646).

Eine weitere Möglichkeit einer milden Zyklisierung ist die Trimethylsilylierung der Peptidbindung zwischen der N-terminalen Aminosäure und dem folgenden Glycin mit Bistrimethylsilyltrifluoracetamid in Acetonitril (4 Stunden unter Rückfluß) (J. S. Davies, R. K. Merritt und R. C. Treadgold, J. Chem. Soc. Perkin Trans. I (1982) 2939 -2947).

Die Guanylierung der Aminofunktion kann mit folgenden Reagentien durchgeführt werden:

1. O-Methylisoharnstoff (S. Weiss und H. Krommer, Chemiker-Zeitung 98 (1974) 617 - 618),

2. S-Methylisothioharnstoff (R. F. Borne, M. L. Forrester und I. W. Waters, J. Med. Chem. 20 (1977) 771 - 776),

3. Nitro-S-Methylisothioharnstoff (L. S. Hafner und R. E. Evans, J. Org. Chem. 24 (1959) 1157),

4. Formamidinosulfonsäure (K. Kim, Y.-T. Lin und H. S. Mosher, Tetrah. Lett. 29 (1988) 3183 - 3186),

5. 3.5-Dimethyl-1-pyrazolyl-formamidinium-nitrat (F. L. Scott, D. G. O'Donovan und J. Reilly, J. Amer. Chem. Soc. 75 (1953) 4053 - 4054).

6. N,N'-Di-tert.-butyloxycarbonyl-S-methyl-isothioharnstoff (R. J. Bergeron und J. S. McManis, J. Org. Chem. 52 (1987) 1700- 1703).

7. N-Alkoxycarbonyl-, N,N'-Dialkoxycarbonyl-, N-Alkylcarbonyl- und N,N'-Dialkylcarbonyl-S-methyl-isothioharnstoff (H. Wollweber, H. Kölling, E. Niemers, A. Widding, P. Andrews, H.-P. Schulz und H. Thomas, Arzneim. Forsch./ Drug Res. 34 (1984) 531 - 542).

Formamidine können aus den entsprechenden Cyanoverbindungen durch Anlagerung von Alkoholen (z. B. Methanol oder Ethanol) in saurem wasserfreiem Medium (z. B. Dioxan, Methanol oder Ethanol) und anschließender Behandlung mit Ammoniak in Alkoholen (z. B. Isopropanol, Methanol oder Ethanol) hergestellt werden (G. Wagner, P. Richter und Ch. Garbe, Pharmazie 29 (1974) 12 - 55). Eine weitere Methode, Formamidine herzustellen, ist die Anlagerung von $H_2S$ an die Cyanogruppe, gefolgt von einer Methylierung des enstandenen Thioamids und anschließender Umsetzung mit Ammoniak (DDR-Patent Nr. 235 866).

Die Ausgangspeptide der allgemeinen Formel IV werden in der Regel vom C-terminalen Ende her stufenweise aufgebaut. Peptidknüpfungen können mit den bekannten Kupplungsmethoden der Peptidchemie durchgeführt werden.

Die folgenden Ausführungen, betreffend die pharmakologischen Eigenschaften der erfindungsgemäßen Imidazolidin-Derivate, ihre Anwendung als Heilmittel in der Therapie und Prophylaxe von Erkrankungen, sie enthaltende pharmazeutische Präparate und deren Herstellung, gelten nicht nur für Verbindungen der allgemeinen Formel i, wie sie zu Beginn der Beschreibung als Gegenstand der vorliegenden Erfindung definiert sind, sondern ebenso auch für diejenigen Verbindungen der allgemeinen Formel i, die bereits als Synthese-Zwischenprodukte, aber noch nicht als pharmakologische Wirkstoffe bekannt waren, und die daher in der für den Stoffschutz gültigen, zu Beginn der Beschreibung gegebenen Definition des Gegenstandes der vorliegenden Erfindung explizit ausgeschlossen wurden. Hinsichtlich der Anwendung und der pharmazeutischen Präparate sind also unter dem Begriff "Verbindungen der allgemeinen Formel I" oder unter ähnlichen Begriffen Verbindungen der allgemeinen Formel I zu verstehen,

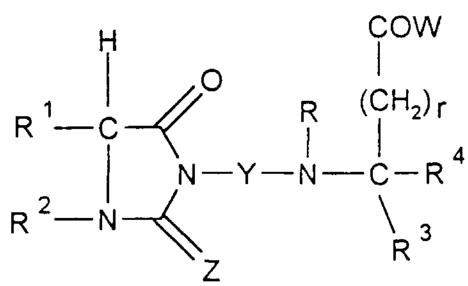

(I)

worin

Y $-(CH_2)_m-CO-$, wobei m für eine ganze Zahl von 1 bis 4 steht, oder

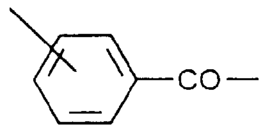

bedeutet;
r eine Zahl von 0 bis 3 bedeutet;
Z Sauerstoff oder Schwefel bedeutet;
W $(C_1-C_{28})$-Alkoxy, $(C_6-C_{14})$-Aryl-$(C_1-C_8)$-alkoxy, $(C_6-C_{14})$-Aryloxy, Amino oder Mono- oder Di-$(C_1-C_{18})$-alkylamino bedeutet;
R Wasserstoff oder $(C_1-C_6)$-Alkyl bedeutet;
$R^1$ $-(CH_2)_n-NH-X$, wobei n für eine ganze Zahl von 1 bis 6 steht,
$-(CH_2)_p-C_6H_4-(CH_2)_q-NH-X$ oder $-(CH_2)_p-C_6H_4-(CH_2)_q-C(=NX^2)-NH_2$, wobei p für eine ganze Zahl von 1 bis 3 und q für eine Zahl von 0 bis 2 steht, bedeutet, wobei aber anstelle von
$>CH-R^1$ auch $>C=CH-C_6H_4-X^1$ stehen kann;
X Wasserstoff, $(C_1-C_6)$-Alkyl, $(C_1-C_6)$-Alkoxycarbonyl, $(C_1-C_6)$-Alkylcarbonyl, $(C_1-C_{18})$-Alkylcarbonyloxy-$(C_1-C_6)$-alkoxycarbonyl, $(C_6-C_{14})$-Aryloxycarbonyl, $(C_6-C_{14})$-Aryl-$(C_1-C_6)$-alkoxycarbonyl, Cyano, Hydroxy, $(C_1-C_6)$-Alkoxy, Amino oder einen Rest der Formel II

$$R'-NH-\underset{|}{C}=N-R'' \qquad (II)$$

bedeutet, worin R' und R" unabhängig voneinander für Wasserstoff, $(C_1-C_6)$-Alkyl, $(C_1-C_6)$-Alkoxycarbonyl, $(C_1-C_6)$-Alkylcarbonyl, $(C_1-C_{18})$-Alkylcarbonyloxy-$(C_1-C_6)$-alkoxycarbonyl, $(C_6-C_{14})$-Aryloxycarbonyl, $(C_6-C_{14})$-Aryl-$(C_1-C_6)$-alkoxycarbonyl, Cyano, Hydroxy, $(C_1-C_6)$-Alkoxy oder Amino stehen;
$X^1$ $-(CH_2)_q-NH-X$ oder $-(CH_2)_q-C(=NX^2)-NH_2$ bedeutet;
$X^2$ Wasserstoff, $(C_1-C_6)$-Alkyl, $(C_1-C_6)$-Alkoxycarbonyl, $(C_1-C_6)$-Alkylcarbonyl, $(C_1-C_{18})$-Alkylcarbonyloxy-$(C_1-C_6)$-alkoxycarbonyl, $(C_6-C_{14})$-Aryloxycarbonyl, $(C_6-C_{14})$-Aryl-$(C_1-C_6)$-alkoxycarbonyl, Cyano, Hydroxy, $(C_1-C_6)$-Alkoxy oder Amino bedeutet;
$R^2$ Wasserstoff oder $(C_1-C_6)$-Alkyl bedeutet;
$R^3$ Wasserstoff oder Phenyl bedeutet;
$R^4$ Wasserstoff, $-COOR^5$, $-CO-N(CH_3)-R^5$ oder $-CO-NH-R^5$ bedeutet;
$R^5$ Wasserstoff oder $(C_1-C_{28})$-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-$(C_1-C_{18})$-alkylaminocarbonyl, Amino-$(C_2-C_{14})$-alkylaminocarbonyl, Amino-$(C_1-C_3)$-alkylphenyl-$(C_1-C_3)$-alkylaminocarbonyl, $(C_1-C_{18})$-Alkylcarbonylamino-$(C_1-C_3)$-alkylphenyl-$(C_1-C_3)$-alkylaminocarbonyl, $(C_1-C_{18})$-Alkylcarbonylamino-$(C_2-C_{14})$-alkylaminocarbonyl, Phenyl-$(C_1-C_8)$-alkoxycarbonyl, Amino, Mercapto, $(C_1-C_{18})$-Alkoxy, $(C_1-C_{18})$-Alkoxycarbonyl, $(C_3-C_8)$-Cycloalkyl, Halogen, Nitro, Trifluormethyl oder einen Rest $R^6$ substituiert ist;
$R^6$ $(C_6-C_{14})$-Aryl, $(C_6-C_{14})$-Aryl-$(C_1-C_8)$-alkyl, einen mono- oder bicyclischen 5- bis 12-gliedrigen heterocyclischen Ring, der aromatisch, teilhydriert oder vollständig hydriert sein kann und der als Heteroelement ein, zwei oder drei gleiche oder verschiedene Stickstoff-, Sauerstoff- oder Schwefelatome enthalten kann, oder einen Rest $R^7$ bedeutet, wobei der Aryl- und unabhängig davon der Heterocyclus-Rest gegebenenfalls ein- oder mehrfach durch gleiche oder verschiedene Reste aus der Reihe $(C_1-C_{18})$-Alkyl, $(C_1-C_{18})$-Alkoxy, Halogen, Nitro oder Trifluormethyl substituiert sein können;
$R^7$ $-NR^8R^9$, $-OR^8$, $-SR^8$, eine Aminosäureseitenkette, einen natürlichen oder unnatürlichen Aminosäure-, Iminosäure- oder Dipeptid-Rest, sowie deren Ester und Amide, wobei freie funktionelle Gruppen durch in der Peptidchemie übliche Schutzgruppen geschützt sein können, oder einen Rest $-COR^{7'}$, worin $R^{7'}$ wie $R^7$ definiert ist, bedeutet;
$R^8$ Wasserstoff, $(C_2-C_{18})$-Alkyl, $(C_6-C_{14})$-Aryl-$(C_1-C_8)$-alkyl, $(C_1-C_{18})$-Alkylcarbonyl, $(C_1-C_{18})$-Alkoxycarbonyl, $(C_6-C_{14})$-Arylcarbonyl, $(C_6-C_{14})$-Aryl-$(C_1-C_8)$-alkylcarbonyl, $(C_6-C_{14})$-Aryl-$(C_1-C_{18})$-alkoxycarbonyl, wobei die Alkylgruppen gegebenenfalls durch eine Aminogruppe substituiert sein können, einen natürlichen oder unnatürlichen

Aminosäure-, Iminosäure- oder einen Dipeptid-Rest bedeutet; und
$R^9$ Wasserstoff, $(C_1$-$C_{18})$-Alkyl, $(C_6$-$C_{14})$-Aryl oder $(C_6$-$C_{14})$-Aryl-$(C_1$-$C_8)$-alkyl bedeutet;

sowie deren physiologisch verträgliche Salze.

Die Verbindungen der allgemeinen Formel I und ihre physiologisch verträglichen Salze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame-Dosis mindestens einer Verbindung der allgemeinen Formel I oder eines Salzes davon, neben üblichen pharmazeutisch einwandfreien Träger- und Zusatzstoffen enthalten. Die Zubereitungen enthalten normalerweise etwa 0,5 bis 90 Gew.% der therapeutisch wirksamen Verbindung.

Die Heilmittel können oral, z. B. in Form von Pillen, Tabletten, Lacktabletten, Dragees, Granulaten, Hart- und Weichgelatinekapseln, Lösungen, Sirupen, Emulsion oder Suspensionen oder Aerosolmischungen verabreicht werden. Die Verabreichung kann aber auch rektal, z. B. in Form von Suppositorien oder parenteral, z. B. in Form von Injektionslösungen, Mikrokapseln oder Rods, perkutan, z. B. in Form von Salben oder Tinkturen, oder nasal, z. B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z. B. Lactose, Maisstärke oder Derivate davon, Talk, Stearinsäure oder deren Salze etc. verwenden. Trägerstoffe für Weichgelatinekapseln und Suppositorien sind z. B. Fette, Wachse, halbfeste und flüssige Polyole, natürliche oder gehärtete Öle etc. Als Trägerstoffe für die Herstellung von Lösungen und Sirupen eignen sich z. B. Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate oder Rods eignen sich Mischpolymerisate aus Glykolsäure und Milchsäure.

Die pharmazeutischen Präparate können neben den Wirk- und Trägerstoffen noch Zusatzstoffe, wie z. B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer physiologisch verträglichen Salze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lantano-Glykoside; Coronardilatatoren, wie Carbochromen; Dipyridamol, Nifedipin und Perhexilin; antianginöse Verbindungen, wie Isosorbiddinitrat, Isosorbidmononitrat, Glycerolnitrat, Molsidomin und Verapamil; β-Blocker, wie Propranolol, Oxprenolol, Atenolol, Metoprolol und Penbutolol. Darüberhinaus lassen sich die Verbindungen mit anderen nootrop wirksamen Substanzen, wie z. B. Piracetam, oder ZNS-aktiven Substanzen, wie Pirlindol, Sulpirid etc., kombinieren.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Applikation beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z. B. 2, 3 oder 4 Teilverabreichungen aufgeteilt. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,2 bis 50 mg, vorzugsweise 0,5 bis 10 mg Wirkstoff der allgemeinen Formel I oder eines ihrer physiologisch verträglichen Salze pro Dosis.

Die erfindungsgemäßen Verbindungen der Formel I haben die Fähigkeit die Zell-Zell-Adhäsion zu hemmen, die auf der Interaktion von Arg-Gly-Asp-enthaltenden Proteinen, wie Fibronectin, Fibrinogen oder des von Willebrand-Faktors mit den sogenannten Integrinen beruhen. Integrine sind Transmembran-Glykoproteine, Rezeptoren für Arg-Gly-Asp-enthaltende Zellmatrix-Glykoproteine (E. Ruoslahti und M. D. Pierschbacher, Science 238 (1987) 491 - 497; D. R. Phillips, I. F. Charo, L. V. Parise und L. A. Fitzgerald, Blood 71 (1988) 831 - 843). Außerdem hemmen sie die Bindung weiterer adhäsiver Proteine, wie Vitronectin, Kollagen und Laminin an die entsprechenden Rezeptoren auf der Oberfläche verschiedener Zelltypen.

Die erfindungsgemäßen Verbindungen der allgemeinen Formel I hemmen die Thrombozytenaggregation, die Metastasierung von Karzinomzellen sowie die Osteoclastenbindung an die Knochenoberflächen.

Die Hydantoinderivate der allgemeinen Formel I finden akut Anwendung bei Thrombosegefahr und chronisch bei der Prävention der Arteriosklerose und Thrombose, z. B. bei der Therapie und Prophylaxe arterieller Gefäßerkrankungen, wie bei akutem Myokardinfarkt, Sekundärprävention des Myokardinfarkts, Reokklusionsprophylaxe nach Lyse und Dilatation (PTCA), instabiler Angina pectoris, transitorischen ischämischen Attacken, Schlaganfall, koronarer Bypass-Operation einschließlich Reokklusionsprophylaxe bei Bypass, Lungenembolie, peripherer arterieller Verschlußkrankheit, Dissezierendem Aneurysma; bei der Therapie venöser und mikrozirkulatorischer Gefäßerkrankungen, wie tiefer Venenthrombose, disseminenter intravaskulärer Gerinnung, postoperativem und post-partum Trauma, chirurgischem oder infektiösem Schock, Septicämie oder bei Erkrankungen mit hyperreagiblen Thrombozyten, thrombotischer thrombozytopenischer Purpura, Preeklampsie, prämenstruellem Syndrom, Dialyse oder extrakorporaler Zirkulation; eine weitere Anwendung ist während Krebsoperationen und auch prophylaktisch bei Krebs gegeben. Ferner kann Osteoporose durch Hemmung der Osteoclastenbindung an die Knochenoberfläche verhindert werden.

Geprüft werden die Verbindungen vor allem auf ihre hemmende Wirkung bei der Blutplättchenaggregation und der Anhaftung von Fibrinogen an Blutplättchen. Verwendet werden gefiltrierte Blutplättchen aus humanem Spenderblut, die mit ADP oder Thrombin aktiviert werden), sowie auf ihre in vivo-Wirkung zur Hemmung der Thrombozytenaggregation und Thrombosehemmung.

**BEISPIELE**

Die Produkte wurden über Massenspektren und/oder NMR-Spektren identifiziert.

**Beispiel 1**

3-(5-(S)-(3-Methoxycarbonylaminopropyl)-2,4-dioxo-imidazolidin-3-yl)-benzoyl-L-aspartyl(OMe)-L-phenylglycinmethylester

**Beispiel 1a**

N-(S)-(Benzyloxycarbonyl-(3-benzyloxycarbonyl-aminopropyl)-methyl)-,N'-(3-ethoxycarbonylphenyl)-harnstoff

Zu 4,1 g (10,5 mmol) Z-L-Ornithinbenzylester-hydrochlorid und 2 g (10,5 mmol) 3-Isocyanatobenzoesäureethylester in 20 ml Dimethylformamid werden bei Raumtemperatur 1,2 g (10,5 mmol) N-Ethylmorpholin zugetropft. Man rührt 12 h bei Raumtemperatur, 1 h bei 50°C, versetzt mit Kaliumhydrogensulfatlösung und extrahiert mit Essigsäureethylester, trocknet die organische Phase und engt ein. Ausbeute: 4,1 g (71 %)

**Beispiel 1b**

3-(5-(S)-(3-Aminopropyl)-2,4-dioxo-imidazolidin-3-yl)-benzoesäure-hydrochlorid

4,1 g (7,5 mmol) N-(S)-(Benzyloxycarbonyl-(3-benzyloxycarbonyl-aminopropyl)-methyl-,N'-(3-ethoxycarbonylphenyl)-harnstoff werden mit 60 ml 6 N Salzsäure 1 h unter Rückfluß erhitzt. Dann wird eingeengt, mit Essigsäureethylester verrührt und abgesaugt.
Ausbeute: 2,1 g (89 %)
Schmelzpunkt: 193 - 196°C

**Beispiel 1c**

3-(5-(S)-(3-Methoxycarbonylaminopropyl)-2,4-dioxo-imidazolidin-3-yl)-benzoesäure

314 mg (1 mmol) 3-(5-(S)-(3-Aminopropyl)-2,4-dioxo-imidazolidin-3-yl)-benzoesäure-hydrochlorid werden in 5 ml Methanol und 5 ml Wasser gelöst. Nach Zugabe von 130 mg (1,38 mmol) Chlorameisensäuremethylester wird bei 10°C mit 0,1 N Natronlauge auf pH 7 eingestellt. Man läßt auf Raumtemperatur erwärmen, rührt 4 h, säuert mit Kaliumhydrogensulfatlösung auf pH 3 an und engt ein. Der Rückstand wird mit Wasser verrührt, abgesaugt und getrocknet.
Ausbeute: 187 mg
Schmelzpunkt 192 - 194°C

**Beispiel 1d**

3-(5-(S)-(Methoxycarbonylaminopropyl)-2,4-dioxo-imidazolidin-3-yl)-benzoyl-L-aspartyl(OMe)-L-phenylglycinmethylester

Zu 87,5 mg (0,26 mmol) 3-(5-(S)-Methoxycarbonylaminopropyl)-2,4-dioxo-imidazolidin-3-yl)-benzoesäure, 106,2 mg (0,26 mmol) H-Asp(OMe)-Phg-OMe-trifluoracetat und 35,1 mg (0,26 mmol) Hydroxybenzotriazol in 10 ml Dimethylformamid gibt man bei 0°C 35 µl (0,28 mmol) N-Ethylmorpholin und 58 mg (0,28 mmol) DCC. Man rührt 1 h bei 0°C, 20 h bei Raumtemperatur, filtriert ausgefallenen Harnstoff ab und engt ein. Der Rückstand wird in Essigsäureethylester aufgenommen und mit Natriumhydrogencarbonatlösung und Kaliumhydrogensulfatlösung gewaschen, die organische Phase getrocknet und eingeengt. Das Produkt wird zur Reinigung über Kieselgel chromatographiert.
Ausbeute: 130 mg
FAB-MS: 611 $(M + H)^+$

**Beispiel 2**

3-(5-(S)-(3-Aminopropyl)-2,4-dioxo-imidazolidin-3-yl)benzoyl-L-aspartyl(OMe)-L-phenylglycinmethylester

**Beispiel 2a**

3-(5-(S)-(3-t-Butoxycarbonylaminopropyl)-2,4-dioxo-imidazolidin-3-yl)-benzoesäure

Zu 9 g (41 mmol) Pyrokohlensäure-di-tert. butylester und 9,1 g (29 mmol) 3-(5-(S)-(3-Aminopropyl)-2,4-dioxo-imidazolidin-3-yl)-benzoesäure-hydrochlorid in 40 ml Dimethylformamid werden bei Raumtemperatur 6,4 g (56 mmol) N-Ethylmorpholin zugetropft. Man rührt 15 h bei Raumtemperatur, filtriert vom Salz ab, engt ein, versetzt mit Kaliumhydrogensulfatlösung und extrahiert mit Essigsäureethylester. Die organische Phase wird mit Tert.-butyl-methylester verrührt und abgesaugt.
Ausbeute: 5,9 g
Schmelzpunkte 174 - 177°C

**Beispiel 2b**

3-(5-(S)-(3-t-Butoxycarbonylaminopropyl)-2,4-dioxo-imidazolidin-3-yl)-benzoyl-L-aspartyl(OMe)-L-phenylglycinmethylester

Zu 300 mg (0,8 mmol) 3-(5-(S)-(3-t-Butoxycarbonylaminopropyl)-2,4-dioxo-imidazolidin-3-yl)-benzoesäure, 330 mg (0,8 mmol) H-Asp(OMe)-Phg-OMe-trifluoracetat und 110 mg (0,8 mmol) Hydroxybenzotriazol in 15 ml Dimethylformamid gibt man bei 0°C 0,1 ml (0,8 mmol) N-Ethylmorpholin und 180 mg (0,87 mmol) DCC. Man rührt 1 h bei 0°C, 20 h bei Raumtemperatur, filtriert vom ausgefallenen Harnstoff ab und engt ein. Der Rückstand wird in Essigsäureethylester aufgenommen und mit Natriumhydrogencarbonatlösung und Kaliumhydrogensulfatlösung gewaschen, die organische Phase getrocknet und eingeengt. Das Produkt wird zur Reinigung über Kieselgel chromatographiert.
Ausbeute: 490 mg

**Beispiel 2c**

3-(5-(S)-(3-Aminopropyl)-2,4-dioxo-imidazolidin-3-yl)benzoyl-L-aspartyl(OMe)-L-phenylglycinmethylester

490 mg (0,75 mmol) 3-(5-(S)-(3-t-Butoxycarbonylaminopropyl)-2,4-dioxo-imidazolidin-3-yl)-benzoyl-L-aspartyl (OMe) -L-phenylglycinmethylester werden mit 0,7 mol 90 %iger Trifluoressigsäure und 10 ml Methylenchlorid versetzt. Man rührt 20 h bei Raumtemperatur, engt im Hochvakuum ein und chromatographiert den Rückstand zur Reinigung an Sephadex LH20 mit einer homogenen Mischung von Butanol/ Eisessig/Wasser.
Ausbeute: 370 mg (74 %)
Schmelzpunkt: 40 - 45°C

Die folgenden Verbindungen können analog den Beispielen 1 und 2 hergestellt werden.

**Beispiel 3**

3-(5-(S)-(3-Acetylaminopropyl)-2,4-dioxo-imidazolidin-3-yl)-benzoyl-L-aspartyl(OMe)-L-phenylglycin-methylester

1,50 g (4,70 mmol) 3-(5-(S)-(3-Acetamidopropyl-2,4-dioxoimidazolidin-3-yl)-benzoesäure und 1,92 g (4,70 mmol) L-Aspartyl(OMe)-L-phenylglycin-OMe werden in 70 ml DMF gelöst und bei 0°C unter Rühren mit 1,07 g (5,17 mmol) Dicyclohexyl-carbodiimid, 0,63 g (4,70 mmol) Hydroxybenzotriazol und 0,6 ml (4,70 mmol) N-Ethylmorpholin versetzt. Nach 24 h wird abfiltriert und im Vakuum eingeengt. Der Rückstand wird in Ethylacetat aufgenommen, mit gesättigter Natriumhydrogencarbonat-Lösung und Wasser gewaschen, getrocknet und am Rotationsverdampfer eingeengt. Der Rückstand wird über Kieselgel chromatographiert

(Dichlormethan/Methanol 98:2 und 95:5). Die Fraktionen, die Substanz enthalten, werden gefriergetrocknet.
Ausbeute: 0,65 g (23%)
Schmelzpunkt: 60°-75°C
$(\alpha)_D^{20}$=+4,35°(c = 0,92, Methanol)
FAB-MS: 592.2 $[M+H]^+$

**Beispiel 4**

(5-(R,S)-(4-Formamidinobenzyl)-2,4-dioxo-imidazolidin-3-yl)-acetyl-L-aspartyl(OBz)-L-valinbenzylester

**Beispiel 5**

(5-(S)-(4-Aminobutyl)-2,4-dioxo-imidazolidin-3-yl)-acetyl-L-aspartyl(OEt)-L-lysinethylester

**Beispiel 6**

(5-(4-Isopropyloxycarbonylaminomethylbenzyliden)-2,4-diox o-imidazolidin-3-yl)-acetyl-L-aspartyl(O-iPr)-L-valinmethylester

**Beispiel 7**

(5-(S)-(3-Di-methoxycarbonylguanidinopropyl)-4-oxo-2-thioxo-imidazolidin-3-yl)-acetyl-L-aspartyl(OMe)-L-phenyl - glycinisopropylester

**Beispiel 8**

(5-(4-Ethoxycarbonylformamidinobenzyliden)-1-methyl-2,4-dioxo-imidazolidin-3-yl)-acetyl-L-aspartyl(OEt)-L-phenylglycinethylester

**Beispiel 9**

(5-(R,S)-(4-Formamidinobenzyl)-2,4-dioxo-imidazolidin-3-yl)-acetyl-L-aspartyl(OMe)-L-phenylglycinmethylester

**Beispiel 10**

(5-(4-Acetylformamidinobenzyliden)-2,4-dioxoimidazolidin-3-yl)-acetyl-L-aspartyl(OMe)-L-phenylglycinmethylester

**Beispiel 11**

(5-(4-Methoxycarbonylformamidinobenzyl)-2,4-dioxo-imidazolidin-3-yl)-acetyl-L-aspartyl(OMe)-L-phenylglycin-methylester

**Beispiel 12**

(5-(4-Aminomethylbenzyliden)-2,4-dioxo-imidazolidin-3-yl)-acetyl-L-aspartyl(OtBu)-L-phenylglycin-methylester

**Beispiel 13**

(5-(S)-(4-Guanidinobenzyl)-2,4-dioxo-imidazolidin-3-yl)-acetyl-L-aspartyl(OMe)-L-phenylglycin-methylester

**Beispiel 14**

a) (5-(S,R)-(4-Formamidino-benzyl)-2,4-dioxoimidazolidin-3-yl)-acetyl-L-aspartyl(O-isopropyl)-L-phenylglycintert.-butylester-acetat

Zu einer Suspension von 1.6 g (4 mmol) H-L-Aspartyl-(O-isopropyl)-L-phenylglycin-tert.-butylester-hydrochlorid, 1.16 g (5-(S,R)-(4-Formamidino-benzyl)-2,4-dioxoimidazolidin-3-yl)-essigsäure und 540 mg HOBt in 25 ml Dimethylformamid gibt man bei 0°C 880 mg Dicyclohexylcarbodiimid. Man rührt 1 Stunde bei °C und 3 Stunden bei Raumtemperatur. Danach wird der Niederschlag abgesaugt und das Filtrat eingeengt und der Rückstand an Kieselgel in einer Mischung aus Methylenchlorid, Methanol, Wasser und Essigsäure wie 85:15:2:2 chromatographiert. Nach dem Einengen wird der Rückstand gefriergetrocknet.
Ausbeute: 2.29 g
$(\alpha)_D^{23}$= -0.8° (c= 1, Methanol)

**Beispiel 15**

(5-(S,R)-(4-Formamidino-benzyl)-2,4-dioxoimidazolidin-3-yl)-acetyl-L-aspartyl(O-isopropyl)-L-phenylglycin

1.2 g (5-(S,R)-(4-Formamidino-benzyl)-2,4-dioxoimidazolidin-3-yl)-acetyl-L-aspartyl(O-isopropyl)-L-phenylglycin-tert.-butylester-acetat werden in 12 ml 90%-iger wässriger Trifluoressigsäure gelöst. Man läßt eine Stunde bei Raumtemperatur stehen, engt ein. Der Rückstand läßt sich nicht zwischen Wasser und Ether verteilen. Die unlösliche Substanz wird abgesaugt und mit Ether gewaschen.
Ausbeute: 560 mg
$(\alpha)_D^{22}$= -9.8° (c= 1, Methanol)

**Beispiel 16**

(5-(S,R)-(4-Formamidino-benzyl)-2,4-dioxoimidazolidin-3-yl)-acetyl-L-aspartyl(O-tert.butyl)-L-phenylglycinisopropylester-acetat

Zu einer Suspension von 3.03 g (5-(S,R)-(4-Formamidino-benzyl)-2,4-dioxoimidazolidin-3-yl)-essigsäure, 4.18 g H-L-Aspartyl(O-tert.-butyl)-L-phenylglycin-isopropylester-hydrochlorid und 1.4 g HOBt in 25 ml Dimethylformamid gibt man bei 0°C 2.29 g Dicyclohexylcarbodiimid. Man rührt 1 Stunde bei °C und 5 Stunden bei Raumtemperatur. Danach wird der Niederschlag abgesaugt und das Filtrat eingeengt und der Rückstand an Kieselgel in einer Mischung aus Methylenchlorid, Methanol, Wasser und Essigsäure wie 85:15:2:2 chromatographiert. Nach dem Einengen wird der Rückstand gefriergetrocknet.
Ausbeute: 4.9 g
$(\alpha)_D^{22}$= -0.4° (c= 1, Methanol)

**Beispiel 17**

(5-(S,R)-(4-Formamidino-benzyl)-2,4-dioxoimidazolidin-3-yl)-acetyl-L-aspartyl-L-phenylglycin-diisopropylester-acetat

Zu einer Suspension von 580.6 mg (5-(S,R)-(4-Formamidinobenzyl)-2,4-dioxoimidazolidin-3-yl)-essigsäure, 773.8 mg H-L-Aspartyl-L-phenylglycin-diisopropylester-hydrochlorid und 270 mg HOBt in 25 ml Dimethylformamid gibt man bei 0°C 440 mg Dicyclohexylcarbodiimid. Man rührt 1 Stunde bei °C und 5 Stunden bei Raumtemperatur. Danach wird der Niederschlag abgesaugt und das Filtrat eingeengt und der Rückstand an Kieselgel in einer Mischung aus Methylenchlorid, Methanol, Wasser und Essigsäure wie 85:15:2:2 chromatographiert. Nach dem Einengen wird der Rückstand gefriergetrocknet.
Ausbeute: 1.04 g
$(\alpha)_D^{24}$= -4.0° (c= 1, Methanol)

**Beispiel 18**

3-(5-(S)-(3-(1-Acetoxyethoxy-carbonyl-amino)-propyl)-2,4-dioxo-imidazolidin-3-yl)-benzoyl-L-aspartyl(OMe)-L-phenylglycin-methylester

a) 3-(5-(S)-(3-(1-Acetoxyethoxy-carbonyl-amino)-propyl) -2,4-dioxo-imidazolidin-3-yl)-benzoesäure
2,50 g (7,97 mmol) 3-[5-(S)-3-Aminopropyl)-2,4-dioxo-imidazolidin-3-yl)-benzoesäure und 2,15 g (7,97 mmol) (1-Acetoxyethyl)-(p-nitrophenyl)-carbonat werden in 50 ml DMF gelöst, mit 3,33 ml (24 mmol) Triethylamin versetzt und 3 d bei Raumtemperatur gerührt. Es wird im Vakuum eingeengt und der Rückstand über Kieselgel chromatographiert (Dichlormethan/Methanol/Eisessig). Ausbeute: 1,95 g (60%) Oel

b) 3-(5-(S)-(3-(1-Acetoxyethoxy-carbonyl-amino)-propyl) -2,4-dioxo-imidazolidin-3-yl)-benzoyl-L-aspartyl(OMe)-L-phenylglycin-methylester

0,42 g (1,03 mmol) 3-(5-(S)-(3-(1-Acetoxyethoxy-carbonyl -amino)-propyl)-2,4-dioxo-imidazolidin-3-yl)-benzoesäure und 0,42 g (1,03 mmol) L-Aspartyl(OMe)-L-phenylglycin-OMe -trifluoracetat werden in 15 ml DMF gelöst und bei 0°C unter Rühren mit 0,23 g (1,13 mmol) Dicyclohexylcarbodiimid, 0,14 g (1,03 mmol) Hydroxybenzotriazol und 0,13 ml (1,03 mmol) N-Ethylmorpholin versetzt. Nach 24 h wird abfiltriert und im Vakuum eingeengt. Der Rückstand wird in Ethylacetat aufgenommen, mit gesättigter Natriumhydrogencarbonat-Lösung und Wasser gewaschen, getrocknet und am Rotationsverdampfer eingeengt. Der Rückstand wird über Kieselgel chromatographiert (Dichlormethan/Methanol 95:5). Die Fraktionen, die Substanz enthalten, werden im Vakuum eingeengt.

Ausbeute: 0,22 g (32%)

Schmelzpunkt: 60°-66°C

$(\alpha)_D^{20}$ = +21,58° (c= 0,695, Methanol)

FAB-MS: 684.2 $[M+H]^+$

**Beispiel 19**

3-[5-(S)-(3-Acetylamino-propyl)-2,4-dioxo-imidazolidin -3-yl)-benzoyl-L-aspartyl-L-phenylglycin-diisopropylester

FAB-MS: 652.3 $[M+H]^+$

**Beispiel 20**

3-[5-(S)-(Guanidinomethyl)-2,4-dioxo-imidazolidin-3-yl)-benzoyl-L-aspartyl-L-phenylglycin-diisopropylester

FAB-MS: 624.2 $[M+H]^+$

**Beispiel 21**

3-[5-(S)-(Methoxycarbonylguanidinomethyl)-2,4-dioxoimidazolidin-3-yl)-benzoyl-L-aspartyl-L-phenylglycindiisopropylester

FAB-MS: 682.3 $[M+H]^+$

Beispiel A

Emulsionen mit 3 mg Wirkstoff per 5 ml können nach folgender Rezeptur hergestellt werden:

| | |
|---|---|
| Wirkstoff | 0,06 g |
| Neutralöl | q. s. |
| Natriumcarboxymethylzellulose | 0,6 g |
| Polyoxyethylenstearat | q. s. |
| Reinglycerin | 0,6 bis 2 g |
| Aromastoffe | q. s. |
| Wasser (entmineralisiert oder destilliert) | ad 100 ml |

Beispiel B

Tabletten können nach folgender Formulierung hergestellt werden:

| | |
|---|---|
| Wirkstoff | 2 mg |
| Lactose | 60 mg |
| Maisstärke | 30 mg |
| lösliche Stärke | 4 mg |
| Magnesiumstearat | 4 mg |
| | 100 mg |

Beispiel C

Für die Herstellung von Weichgelatinekapseln mit 5 mg Wirkstoff pro Kapsel eignet sich die folgende Zusammensetzung:

| | |
|---|---|
| Wirkstoff | 5 mg |
| Mischung von Triglyceriden aus Kokosöl | 150 mg |
| Kapselinhalt | 155 mg |

Beispiel D

Für die Herstellung von Dragees eignet sich folgende Formulierung:

| | |
|---|---|
| Wirkstoff | 3 mg |
| Maisstärke | 100 mg |
| Lactose | 55 mg |
| sec. Calciumphosphat | 30 mg |
| lösliche Stärke | 3 mg |
| Magnesiumstearat | 5 mg |
| kolloidale Kieselsäure | 4 mg |
| | 200 mg |

Beispiel E

Dragees, enthaltend einen erfindungsgemäßen Wirkstoff und einen anderen therapeutisch wirksamen Stoff:

| | |
|---|---|
| Wirkstoff | 6 mg |
| Propanolol | 40 mg |
| Milchzucker | 90 mg |
| Maisstärke | 90 mg |
| sec. Calciumphosphat | 34 mg |
| lösliche Stärke | 3 mg |
| Magnesiumstearat | 3 mg |
| kolloidale Kieselsäure | 4 mg |
| | 270 mg |

Beispiel F

Dragees, enthaltend einen erfindungsgemäßen Wirkstoff und einen anderen therapeutisch wirksamen Stoff:

| | |
|---|---|
| Wirkstoff | 5 mg |
| Pirlindol | 5 mg |
| Milchzucker | 60 mg |
| Maisstärke | 90 mg |
| sec. Calciumphosphat | 30 mg |
| lösliche Stärke | 3 mg |
| Magnesiumstearat | 3 mg |
| kolloidale Kieselsäure | 4 mg |
| | 200 mg |

Beispiel G

Kapseln, enthaltend einen erfindungsgemäßen Wirkstoff und einen anderen therapeutisch wirksamen Stoff:

| | |
|---|---|
| Wirkstoff | 5 mg |
| Nicergolin | 5 mg |
| Maisstärke | 185 mg |
| | 195 mg |

Beispiel H

Injektionslösungen mit 1 mg Wirkstoff pro ml können nach folgender Rezeptur hergestellt werden:

| | |
|---|---|
| Wirkstoff | 1,0 mg |
| Polyethylenglykol 400 | 0,3 mg |
| Natriumchlorid | 2,7 mg |
| Wasser zu Injektionszwecken auf | 1 ml |

**Patentansprüche**

**1.** Verbindungen der allgemeinen Formel I

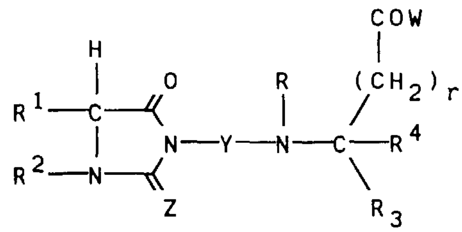

(I)

worin

**Y** $-(CH_2)_m-CO-$, wobei m für eine ganze Zahl von 1 bis 4 steht, oder

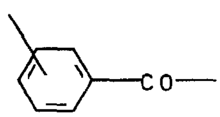

bedeutet;

**r** eine Zahl von 0 bis 3 bedeutet;

**Z** Sauerstoff oder Schwefel bedeutet;

**W** $(C_1-C_{28})$-Alkoxy, $(C_6-C_{14})$-Aryl-$(C_1-C_8$-alkoxy, $(C_6-C_{14})$-Aryloxy, Amino oder Mono- oder Di-$(C_1-C_{18})$-alkylamino bedeutet;

**R** Wasserstoff oder $(C_1-C_6)$-Alkyl bedeutet;

**$R^1$** $-(CH_2)_n-NH-X$, wobei n für eine ganze Zahl von 1 bis 6 steht, $-(CH_2)_p-C_6H_4-(CH_2)_q-NH-X$ oder $-(CH_2)_p-C_6H_4-(CH_2)_q-C(=NX^2)-NH_2$, wobei p für eine ganze Zahl von 1 bis 3 und q für eine Zahl von 0 bis 2 steht, bedeutet, wobei aber anstelle von
$>CH-R^1$ auch $>C=CH-C_6H_4-X^1$ stehen kann ;

**X** Wasserstoff, $(C_1-C_6)$-Alkyl, $(C_1-C_6)$-Alkoxycarbonyl, $(C_1-C_6)$-Alkylcarbonyl, $(C_1-C_{18})$-Alkylcarbonyloxy-$(C_1$-

$C_6$)-alkoxycarbonyl, ($C_6$-$C_{14}$)-Aryloxycarbonyl, ($C_6$-$C_{14}$)-Aryl-($C_1$-$C_6$)-alkoxycarbonyl, Cyano, Hydroxy, ($C_1$-$C_6$)-Alkoxy, Amino oder einen Rest der Formel II

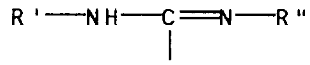
(II)

bedeutet, worin R' und R" unabhängig voneinander für Wasserstoff, ($C_1$-$C_6$)-Alkyl, ($C_1$-$C_6$)-Alkoxycarbonyl, ($C_1$-$C_6$)-Alkylcarbonyl, ($C_1$-$C_{18}$)-Alkylcarbonyloxy-($C_1$-$C_6$)-alkoxycarbonyl, $C_6$-$C_{14}$)-Aryloxycarbonyl, ($C_6$-$C_{14}$)-Aryl-($C_1$-$C_6$)-alkoxycarbonyl, Cyano, Hydroxy, ($C_1$-$C_6$)-Alkoxy oder Amino stehen;

**$X^1$** $-(CH_2)_q$-NH-X oder $-(CH_2)_q-C(=NX^2)-NH_2$ bedeutet;

**$X^2$** Wasserstoff, ($C_1$-$C_6$)-Alkyl, ($C_1$-$C_6$)-Alkoxycarbonyl, ($C_1$-$C_6$)-Alkylcarbonyl, ($C_1$-$C_{18}$) -Alkylcarbonyloxy-($C_1$-$C_6$)-alkoxycarbonyl, ($C_6$-$C_{14}$)-Aryloxycarbonyl, ($C_6$-$C_{14}$)-Aryl-($C_1$-$C_6$)-alkoxycarbonyl, Cyano, Hydroxy, ($C_1$-$C_6$)-Alkoxy, oder Amino bedeutet;

**$R^2$** Wasserstoff oder ($C_1$-$C_6$)-Alkyl bedeutet;

**$R^3$** Wasserstoff oder Phenyl bedeutet;

**$R^4$** Wasserstoff, $-COOR^5$, $-CO-N(CH_3)-R^5$ oder $-CO-NH-R^5$ bedeutet;

**$R^5$** Wasserstoff oder ($C_1$-$C_{28}$)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-($C_1$-$C_{18}$)-Alkylaminocarbonyl, Amino-($C_2$-$C_{14}$)-alkylaminocarbonyl, Amino-($C_1$-$C_3$)alkylphenyl-($C_1$-$C_3$)alkylaminocarbonyl, ($C_1$-$C_{18}$)-Alkylcarbonylamino-($C_1$-$C_3$)-alkylphenyl-($C_1$-$C_3$)alkylaminocarbonyl, ($C_1$-$C_{18}$)-Alkylcarbonylamino-($C_2$-$C_{14}$)-alkylaminocarbonyl, Phenyl-($C_1$-$C_8$)-alkoxycarbonyl, Amino, Mercapto, ($C_1$-$C_{18}$)-Alkoxy, ($C_1$-$C_{18}$)-Alkoxycarbonyl, ($C_3$-$C_8$)-Cycloalkyl, Halogen, Nitro, Trifluormethyl oder einen Rest $R^6$ substituiert ist;

**$R^6$** ($C_6$-$C_{14}$)-Aryl, ($C_6$-$C_{14}$)-Aryl-($C_1$-$C_8$)-Alkyl, einen mono- oder bicyclischen 5- bis 12-gliedrigen heterocyclischen Ring, der aromatisch, teilhydriert oder vollständig hydriert sein kann und der als Heteroelement ein, zwei oder drei gleiche oder verschiedene Stickstoff-, Sauerstoff- oder Schwefel-Atome enthalten kann, oder einen Rest $R^7$ bedeutet, wobei der Aryl- und unabhängig davon der Heterocyclus-Rest gegebenenfalls ein- oder mehrfach durch gleiche oder verschiedene Reste aus der Reihe ($C_1$-$C_{18}$)-Alkyl, ($C_1$-$C_{18}$)-Alkoxy, Halogen, Nitro oder Trifluormethyl substituiert sein können;

**$R^7$** $-NR^8R^9$, $-OR^8$, $-SR^8$, eine Aminosäureseitenkette, einen natürlichen oder unnatürlichen Aminosäure-, Iminosäure- oder Dipeptid-Rest, sowie deren Ester und Amide, wobei freie funktionelle Gruppen durch in der Peptidchemie übliche Schutzgruppen geschützt sein können, oder einen Rest $-COR^{7'}$, worin $R^{7'}$ wie $R^7$ definiert ist, bedeutet;

**$R^8$** Wasserstoff, ($C_2$-$C_{18}$)-Alkyl, ($C_6$-$C_{14}$)-Aryl-($C_1$-$C_8$)-alkyl, ($C_1$-$C_{18}$)-Alkylcarbonyl, ($C_1$-$C_{18}$)-Alkoxycarbonyl, ($C_6$-$C_{14}$)-Arylcarbonyl, ($C_6$-$C_{14}$)-Aryl-($C_1$-$C_8$)-alkylcarbonyl, ($C_6$-$C_{14}$)-Aryl-($C_1$-$C_{18}$)-alkoxycarbonyl, wobei die Alkylgruppen gegebenenfalls durch eine Aminogruppe substituiert sein können, einen natürlichen oder unnatürlichen Aminosäure-, Iminosäure- oder einen Dipeptid-Rest bedeutet; und

**$R^9$** Wasserstoff, ($C_1$-$C_{18}$)-Alkyl, ($C_6$-$C_{14}$)-Aryl oder ($C_6$-$C_{14}$)-Aryl-($C_1$-$C_8$)-alkyl bedeutet;

sowie deren physiologisch verträgliche Salze,
wobei Verbindungen der allgemeinen Formel I ausgeschlossen sind, in denen gleichzeitig

Y für $-CH_2-CO-$ steht;
r die Zahl 1 bedeutet;
Z Sauerstoff bedeutet;
W tert.-Butoxy bedeutet;
R, $R^2$ und $R^3$ Wasserstoff bedeuten;

$R^1$ $-(CH_2)_k-NHX^a$ bedeutet, wobei k für 3 oder 4 steht;
$X^a$ Wasserstoff, $(C_1-C_6)$-Alkyl oder einen Rest der Formel

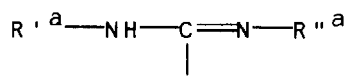

bedeutet, in dem $R'^a$ und $R''^a$ unabhängig voneinander Wasserstoff oder $(C_1-C_6)$-Alkyl bedeuten;
$R^4$ $-CONHR^5$ bedeutet.

**2.** Verbindungen gemäß Anspruch 1, dadurch gekennzeichnet, daß in der allgemeinen Formel I

**Y** $-(CH_2)_m-CO-$, wobei m für 1 oder 2 steht, oder

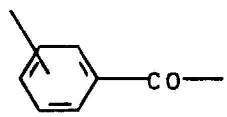

bedeutet;

**r** 1 bedeutet;

**Z** Sauerstoff oder Schwefel bedeutet;

**W** $(C_1-C_4)$-Alkoxy, insbesondere Methoxy, Ethoxy oder 2-Propyloxy, bedeutet;

**R** Wasserstoff bedeutet;

**R¹** $-(CH_2)_n-NH-X$, wobei n für eine ganze Zahl von 1 bis 4 steht, $-CH_2-C_6H_4-(CH_2)_q-NH-X$ oder $-CH_2-C_6H_4-(CH_2)_q-C-(=NX^2)-NH_2$, wobei q für 0 oder 1 steht, bedeutet, wobei aber anstelle von $\rangle CH-R^1$ auch $\rangle C=CH-C_6H_4-X^1$ stehen kann ;

**X** Wasserstoff, $(C_1-C_6)$-Alkoxycarbonyl, $(C_1-C_6)$-Alkylcarbonyl, $(C_6-C_{14})$-Aryl-$(C_1-C_6)$-alkoxycarbonyl oder einen Rest der Formel II

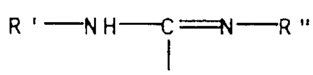

(II)

bedeutet, worin R' und R" unabhängig voneinander für Wasserstoff, $(C_1-C_6)$-Alkoxycarbonyl $(C_1-C_6)$-Alkylcarbonyl oder $(C_6-C_{14})$-Aryl-$(C_1-C_6)$-alkoxycarbonyl stehen;

**X¹** $-(CH_2)_q-NH-X$ oder $-C(=NX^2)-NH_2$, wobei q für 0 oder 1 steht, bedeutet;

**X²** Wasserstoff, $(C_1-C_6)$-Alkoxycarbonyl, $(C_1-C_6)$-Alkylcarbonyl oder $(C_6-C_{14})$-Aryl-$(C_1-C_6)$-alkoxycarbonyl bedeutet;

**R²** Wasserstoff oder Methyl bedeutet;

**R³** Wasserstoff bedeutet; und

**R⁴** $-CO-NH-R^5$ bedeutet, wobei $-NH-R^5$ für einen α-Aminosäurerest, dessen ω-Amino-$(C_2-C_8)$-alkylamid oder dessen $(C_1-C_8)$-Alkyl- oder Benzylester steht.

3. Verbindungen gemäß Anspruch 2, dadurch gekennzeichnet, daß für -NH-$R^5$ stehende α-Aminosäurereste der Valin-, Lysin-, Phenylalanin-, Phenylglycin- oder der 4-Chlorphenylglycin-Rest sind.

4. Verbindungen gemäß Anspruch 2, dadurch gekennzeichnet, daß das ω-Amino-($C_2$-$C_8$)-alkylamid das 4-Aminobutylamid ist.

5. Verfahren zur Herstellung von Verbindungen der Ansprüche 1 bis 4, dadurch gekennzeichnet, daß man eine Fragmentkondensation einer Verbindung der allgemeinen Formel III

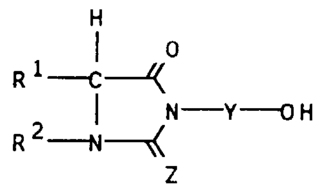

(III)

mit einer Verbindung der allgemeinen Formel IV

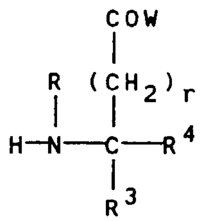

(IV)

wobei die Reste R, $R^1$ bis $R^4$ und r, Y, Z und W wie in Anspruch 1 angegeben definiert sind, ausführt.

6. Verbindungen der allgemeinen Formel I,

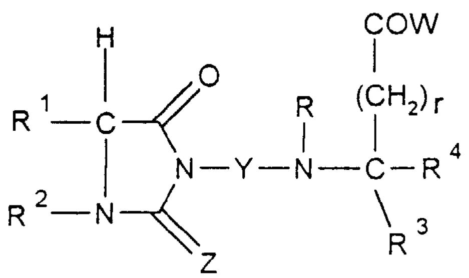

(I)

worin

Y -$(CH_2)_m$-CO-, wobei m für eine ganze Zahl von 1 bis 4 steht, oder

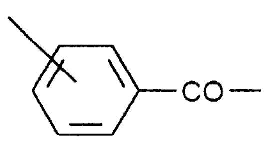

bedeutet;
r eine Zahl von 0 bis 3 bedeutet;
Z Sauerstoff oder Schwefel bedeutet;
W ($C_1$-$C_{28}$)-Alkoxy, ($C_6$-$C_{14}$)-Aryl-($C_1$-$C_8$)-alkoxy, ($C_6$-$C_{14}$)-Aryloxy, Amino oder Mono- oder Di-($C_1$-$C_{18}$)-alkylamino bedeutet;

R Wasserstoff oder ($C_1$-$C_6$)-Alkyl bedeutet;
$R^1$ -$(CH_2)_n$-NH-X, wobei n für eine ganze Zahl von 1 bis 6 steht,-$(CH_2)_p$-$C_6H_4$-$(CH_2)_q$-NH-X oder -$(CH_2)_p$-$C_6H_4$-$(CH_2)_q$-C(=$NX^2$)-$NH_2$, wobei p für eine ganze Zahl von 1 bis 3 und q für eine Zahl von 0 bis 2 steht, bedeutet, wobei aber anstelle von
$>$CH-$R^1$ auch $>$C=CH-$C_6H_4$-$X^1$ stehen kann;
X Wasserstoff, ($C_1$-$C_6$)-Alkyl, ($C_1$-$C_6$)-Alkoxycarbonyl, ($C_1$-$C_6$)-Alkylcarbonyl, ($C_1$-$C_{18}$)-Alkylcarbonyloxy-($C_1$-$C_6$)-alkoxycarbonyl, ($C_6$-$C_{14}$)-Aryloxycarbonyl, ($C_6$-$C_{14}$)-Aryl-($C_1$-$C_6$)-alkoxycarbonyl, Cyano, Hydroxy, ($C_1$-$C_6$)-Alkoxy, Amino oder einen Rest der Formel II

$$R' - NH - \underset{|}{C} = N - R'' \qquad (II)$$

bedeutet, worin R' und R" unabhängig voneinander für Wasserstoff, ($C_1$-$C_6$)-Alkyl, ($C_1$-$C_6$)-Alkoxycarbonyl, ($C_1$-$C_6$)-Alkylcarbonyl, ($C_1$-$C_{18}$)-Alkylcarbonyloxy-($C_1$-$C_6$)-alkoxycarbonyl, ($C_6$-$C_{14}$)-Aryloxycarbonyl, ($C_6$-$C_{14}$)-Aryl-($C_1$-$C_6$)-alkoxycarbonyl, Cyano, Hydroxy, ($C_1$-$C_6$)-Alkoxy oder Amino stehen;
$X^1$ -$(CH_2)_q$-NH-X oder -$(CH_2)_q$-C(=$NX^2$)-$NH_2$ bedeutet;
$X^2$ Wasserstoff, ($C_1$-$C_6$)-Alkyl, ($C_1$-$C_6$)-Alkoxycarbonyl, ($C_1$-$C_6$)-Alkylcarbonyl, ($C_1$-$C_{18}$)-Alkylcarbonyloxy-($C_1$-$C_6$)-alkoxycarbonyl, ($C_6$-$C_{14}$)-Aryloxycarbonyl, ($C_6$-$C_{14}$)-Aryl-($C_1$-$C_6$)-alkoxycarbonyl, Cyano, Hydroxy, ($C_1$-$C_6$)-Alkoxy oder Amino bedeutet; $R^2$ Wasserstoff oder ($C_1$-$C_6$)-Alkyl bedeutet;
$R^3$ Wasserstoff oder Phenyl bedeutet;
$R^4$ Wasserstoff, -$COOR^5$, -CO-N($CH_3$)-$R^5$ oder -CO-NH-$R^5$ bedeutet;
$R^5$ Wasserstoff oder ($C_1$-$C_{28}$)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-($C_1$-$C_{18}$)-alkylaminocarbonyl, Amino-($C_2$-$C_{14}$)-alkylaminocarbonyl, Amino-($C_1$-$C_3$)-alkylphenyl-($C_1$-$C_3$)-alkylaminocarbonyl, ($C_1$-$C_{18}$)-Alkylcarbonylamino-($C_1$-$C_3$)-alkylphenyl-($C_1$-$C_3$)-alkylaminocarbonyl, ($C_1$-$C_{18}$)-Alkylcarbonylamino-($C_2$-$C_{14}$)-alkylaminocarbonyl, Phenyl-($C_1$-$C_8$)-alkoxycarbonyl, Amino, Mercapto, ($C_1$-$C_{18}$)-Alkoxy, ($C_1$-$C_{18}$)-Alkoxycarbonyl, ($C_3$-$C_8$)-Cycloalkyl, Halogen, Nitro, Trifluormethyl oder einen Rest $R^6$ substituiert ist;
$R^6$ ($C_6$-$C_{14}$)-Aryl, ($C_6$-$C_{14}$)-Aryl-($C_1$-$C_8$)-alkyl, einen mono- oder bicyclischen 5- bis 12-gliedrigen heterocyclischen Ring, der aromatisch, teilhydriert oder vollständig hydriert sein kann und der als Heteroelement ein, zwei oder drei gleiche oder verschiedene Stickstoff-, Sauerstoff- oder Schwefelatome enthalten kann, oder einen Rest $R^7$ bedeutet, wobei der Aryl- und unabhängig davon der Heterocyclus-Rest gegebenenfalls ein- oder mehrfach durch gleiche oder verschiedene Reste aus der Reihe ($C_1$-$C_{18}$)-Alkyl, ($C_1$-$C_{18}$)-Alkoxy, Halogen, Nitro oder Trifluormethyl substituiert sein können;
$R^7$ -$NR^8R^9$, -$OR^8$, -$SR^8$, eine Aminosäureseitenkette, einen natürlichen oder unnatürlichen Aminosäure-, Iminosäure- oder Dipeptid-Rest, sowie deren Ester und Amide, wobei freie funktionelle Gruppen durch in der Peptidchemie übliche Schutzgruppen geschützt sein können, oder einen Rest -$COR^{7'}$, worin $R^{7'}$ wie $R^7$ definiert ist, bedeutet;
$R^8$ Wasserstoff, ($C_2$-$C_{18}$)-Alkyl, ($C_6$-$C_{14}$)-Aryl-($C_1$-$C_8$)-alkyl, ($C_1$-$C_{18}$)-Alkylcarbonyl, ($C_1$-$C_{18}$)-Alkoxycarbonyl, ($C_6$-$C_{14}$)-Arylcarbonyl, ($C_6$-$C_{14}$)-Aryl-($C_1$-$C_8$)-alkylcarbonyl, ($C_6$-$C_{14}$)-Aryl-($C_1$-$C_{18}$)-alkoxycarbonyl, wobei die Alkylgruppen gegebenenfalls durch eine Aminogruppe substituiert sein können, einen natürlichen oder unnatürlichen Aminosäure-, Iminosäure- oder einen Dipeptid-Rest bedeutet; und
$R^9$ Wasserstoff, ($C_1$-$C_{18}$)-Alkyl, ($C_6$-$C_{14}$)-Aryl oder ($C_6$-$C_{14}$)-Aryl-($C_1$-$C_8$)-alkyl bedeutet;

oder worin die Reste in der allgemeinen Formel i wie in den Ansprüchen 2 bis 4 angegeben definiert sind, sowie deren physiologisch verträgliche Salze zur Anwendung als Hemmstoffe der Thrombozytenaggregation, der Metastasierung von Karzinomzellen oder der Osteoclastenbindung an die Knochenoberflächen.

**7.** Pharmazeutisches Präparat, dadurch gekennzeichnet, daß es eine oder mehrere der in Anspruch 6 definierten Verbindungen der allgemeinen Formel I oder ein physiologisch verträgliches Salz davon als Wirkstoff zusammen mit pharmazeutisch annehmbaren Träger- und Zusatzstoffen und gegebenenfalls noch einen oder mehrere andere pharmakologische Wirkstoffe enthält.

**8.** Verfahren zur Herstellung eines pharmazeutischen Präparates, enthaltend eine oder mehrere der in Anspruch 6 definierten Verbindungen der allgemeinen Formel I oder ein physiologisch verträgliches Salz davon, dadurch gekennzeichnet, daß man diese zusammen mit pharmazeutisch annehmbaren Träger- und Zusatzstoffen und gegebenenfalls noch einem oder mehreren anderen pharmakologischen Wirkstoffen in eine geeignete Darreichungsform bringt.

## Claims

1. Compounds of the formula I

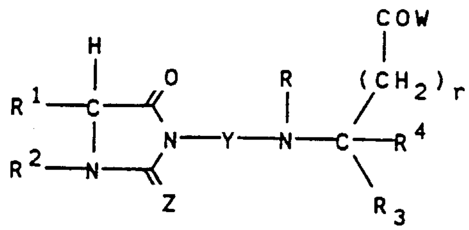

(I)

in which

**Y** denotes $-(CH_2)_m-CO-$, where m represents an integer from 1 to 4, or

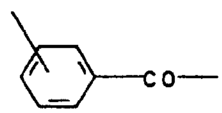

;

**r** denotes a number from 0 to 3;

**Z** denotes oxygen or sulphur;

**W** denotes $(C_1-C_{28})$ -alkoxyl $(C_6-C_{14})$-aryl-$(C_1-C_8)$-alkoxy, $(C_6-C_{14})$-aryloxy, amino or mono- or di-$(C_1-C_{18})$-alkylamino;

**R** denotes hydrogen or $(C_1-C_6)$-alkyl;

**R¹** denotes $-(CH_2)_n-NH-X$, where n represents an integer from 1 to 6, $-(CH_2)_p-C_6H_4-(CH_2)_q-NH-X$ or $-(CH_2)_p-C_6H_4-(CH_2)_q-C(=NX^2)-NH_2$, where p represents an integer from 1 to 3 and q represents a number from 0 to 2, but also where instead of
$>CH-R^1$ $>C=CH-C_6H_4-X^1$ can be present;

**X** denotes hydrogen, $(C_1-C_6)$-alkyl, $(C_1-C_6)$-alkoxycarbonyl, $(C_1-C_6)$ -alkylcarbonyl, $(C_1-C_{18})$ -alkylcarbonyloxy-$(C_1-C_6)$-alkoxycarbonyl, $(C_6-C_{14})$-aryloxycarbonyl, $(C_6-C_{14})$-aryl-$(C_1-C_6)$-alkoxycarbonyl, cyano, hydroxyl, $(C_1-C_6)$-alkoxy, amino or a radical of the formula II

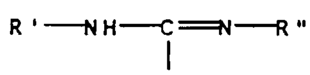

(II)

in which R' and R" independently of one another represent hydrogen, $(C_1-C_6)$-alkyl, $(C_1-C_6)$-alkoxycarbonyl, $(C_1-C_6)$-alkylcarbonyl, $(C_1-C_{18})$-alkylcarbonyloxy-$(C_1-C_6)$-alkoxycarbonyl, $(C_6-C_{14})$-aryloxycarbonyl, $(C_6-C_{14})$-aryl-$(C_1-C_6)$-alkoxycarbonyl, cyano, hydroxyl, $(C_1-C_6)$-alkoxy or amino;

**X¹** denotes $-(CH_2)_q-NH-X$ or $-(CH_2)_q-C(=NX^2)-NH_2$;

**X²** denotes hydrogen, $(C_1-C_6)$-alkyl, $(C_1-C_6)$-alkoxycarbonyl, $(C_1-C_6)$-alkylcarbonyl, $(C_1-C_{18})$-alkylcarbonyloxy-$(C_1-C_6)$-alkoxycarbonyl, $(C_6-C_{14})$-aryloxycarbonyl, $(C_6-C_{14})$-aryl- $(C_1-C_6)$-alkoxycarbonyl, cyano, hydroxyl, $(C_1-C_6)$-alkoxy or amino;

**R²** denotes hydrogen or $(C_1-C_6)$-alkyl;

**R³** denotes hydrogen or phenyl;

**R⁴** denotes hydrogen, $-COOR^5$, $-CO-N(CH_3)-R^5$ or $-CO-NH-R^5$;

**R⁵** denotes hydrogen or $(C_1-C_{28})$-alkyl, which is optionally monosubstituted or polysubstituted by identical or different radicals from the series consisting of hydroxyl, hydroxycarbonyl, aminocarbonyl, mono- or di-$(C_1-C_{18})$ -alkylaminocarbonyl, amino- $(C_2-C_{14})$-alkylaminocarbonyl, amino- $(C_1-C_3)$-alkylphenyl-$(C_1-C_3)$-alkylaminocarbonyl, $(C_1-C_{18})$-alkylcarbonylamino-$(C_1-C_3)$-alkylphenyl-$(C_1-C_3)$-alkylaminocarbonyl, $(C_1-C_{18})$-alkylcarbonylamino-$(C_2-C_{14})$-alkylaminocarbonyl, phenyl-$(C_1-C_8)$-alkoxycarbonyl, amino, mercapto, $(C_1-C_{18})$-alkoxy, $(C_1-C_{18})$-alkoxycarbonyl, $(C_3-C_8)$-cycloalkyl, halogen, nitro, trifluoromethyl or a radical $R^6$;

**R⁶** denotes $(C_6-C_{14})$-aryl, $(C_6-C_{14})$-aryl-$(C_1-C_8)$-alkyl, a monocyclic or bicyclic 5- to 12-membered heterocyclic ring which can be aromatic, partially hydrogenated or completely hydrogenated and which, as the heteroelement, can contain one, two or three identical or different nitrogen, oxygen or sulphur atoms, or denotes a radical $R^7$, where the aryl radical and, independently thereof, the heterocyclic radical can be optionally monosubstituted or polysubstituted by identical or different radicals from the series consisting of $(C_1-C_{18})$-alkyl, $(C_1-C_{18})$-alkoxy, halogen, nitro and trifluoromethyl;

**R⁷** denotes $-NR^8R^9$, $-OR^8$, $-SR^8$, an amino acid side chain, a natural or unnatural amino acid residue, imino acid residue or dipeptide residue, and also their esters and amides, where free functional groups can be protected by protective groups customary in peptide chemistry, or denotes a radical $-COR^{7'}$, in which $R^{7'}$ is defined as $R^7$;

**R⁸** denotes hydrogen, $(C_2-C_{18})$-alkyl, $(C_6-C_{14})$-aryl-$(C_1-C_8)$-alkyl, $(C_1-C_{18})$-alkylcarbonyl, $(C_1-C_{18})$-alkoxycarbonyl, $(C_6-C_{14})$-arylcarbonyl, $(C_6-C_{14})$-aryl-$(C_1-C_8)$-alkylcarbonyl, or $(C_6-C_{14})$-aryl-$(C_1-C_{18})$-alkoxycarbonyl, where the alkyl groups can optionally be substituted by an amino group, denotes a natural or unnatural amino acid residue, imino acid residue or a dipeptide residue; and

**R⁹** denotes hydrogen, $(C_1-C_{18})$-alkyl, $(C_6-C_{14})$-aryl or $(C_6-C_{14})$-aryl-$(C_1-C_8)$-alkyl;

and their physiologically tolerable salts,
where compounds of the formula I are excluded in which simultaneously

Y represents $-CH_2-CO-$;
r denotes the number 1;
Z denotes oxygen;
W denotes tert-butoxy;
R, $R^2$ and $R^3$ denote hydrogen;
$R^1$ denotes $-(CH_2)_k-NHX^a$, where k represents 3 or 4; $X^a$ denotes hydrogen, $(C_1-C_6)$-alkyl or a radical of the formula

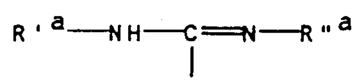

in which $R'^a$ and $R''^a$ independently of one another denote hydrogen or $(C_1-C_6)$-alkyl;
$R^4$ denotes $-CONHR^5$.

**2.** Compounds according to Claim 1, characterised in that in the formula I

**Y** denotes $-(CH_2)_m-CO-$, where m represents 1 or 2, or

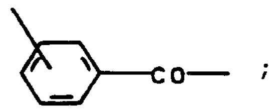

**r** denotes 1;

**Z** denotes oxygen or sulphur;

**W** denotes ($C_1$-$C_4$)-alkoxy, in particular methoxy, ethoxy or 2-propoxy;

**R** denotes hydrogen;

**$R^1$** denotes -$(CH_2)_n$-NH-X, where n represents an integer from 1 to 4, -$CH_2$-$C_6H_4$-$(CH_2)_q$-NH-X or -$CH_2$-$C_6H_4$-$(CH_2)_q$-C-(=$NX^2$)-$NH_2$, where q represents 0 or 1, but also where instead of $>$CH-$R^1$ $>$C=CH-$C_6H_4$-$X^1$ can be present;

**X** denotes hydrogen, ($C_1$-$C_6$)-alkoxycarbonyl, ($C_1$-$C_6$)-alkylcarbonyl, ($C_6$-$C_{14}$)-aryl-($C_1$-$C_6$)-alkoxycarbonyl or a radical of the formula II

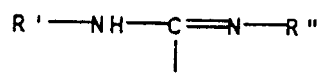 (II)

in which R' and R" independently of one another represent hydrogen, ($C_1$-$C_6$)-alkoxycarbonyl, ($C_1$-$C_6$)-alkylcarbonyl or ($C_6$-$C_{14}$)-aryl-($C_1$-$C_6$)-alkoxycarbonyl;

**$X^1$** denotes -$(CH_2)_q$-NH-X or -C(=$NX^2$)-$NH_2$, where q represents 0 or 1;

**$X^2$** denotes hydrogen, ($C_1$-$C_6$)-alkoxycarbonyl, ($C_1$-$C_6$)-alkylcarbonyl or ($C_6$-$C_{14}$)-aryl-($C_1$-$C_6$)-alkoxycarbonyl;

**$R^2$** denotes hydrogen or methyl;

**$R^3$** denotes hydrogen; and

**$R^4$** denotes -CO-NH-$R^5$, where -NH-$R^5$ represents an α-amino acid residue, its ω-amino-($C_2$-$C_8$)-alkyl amide or its ($C_1$-$C_8$)-alkyl or benzyl ester.

**3.** Compounds according to Claim 2, characterised in that α-amino acid radicals representing -NH-$R^5$ are the valine, lysine, phenylalanine, phenylglycine or the 4-chlorophenylglycine radical.

**4.** Compounds according to Claim 2, characterised in that the ω-amino-($C_2$-$C_8$)-alkyl amide is the 4-aminobutyl amide.

**5.** Process for the preparation of compounds of Claims 1 to 4, characterised in that a fragment condensation of a compound of the formula III

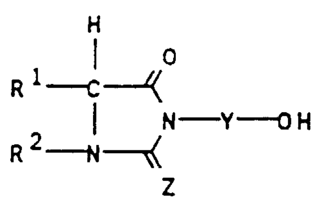 (III)

with a compound of the formula IV

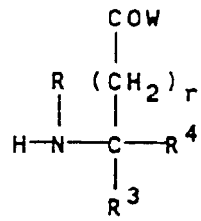

(IV)

where the radicals R, $R^1$ to $R^4$ and r, Y, Z and W are defined as indicated in Claim 1, is carried out.

**6.** Compounds of the formula I

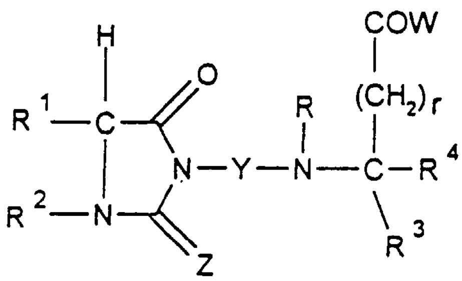

(I)

in which

Y denotes $-(CH_2)_m$-CO-, where m represents an integer from 1 to 4, or

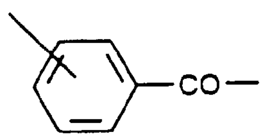 ;

r denotes a number from 0 to 3;
Z denotes oxygen or sulphur;
W denotes ($C_1$-$C_{28}$)-alkoxy, ($C_6$-$C_{14}$)-aryl-($C_1$-$C_8$)-alkoxy, ($C_6$-$C_{14}$)-aryloxy, amino or mono- or di-($C_1$-$C_{18}$)-alkylamino;
R denotes hydrogen or ($C_1$-$C_6$)-alkyl;
$R^1$ denotes $-(CH_2)_n$-NH-X, where n represents an integer from 1 to 6, $-(CH_2)_p$-$C_6H_4$-$(CH_2)_q$-NH-X or $-(CH_2)_p$-$C_6H_4$-$(CH_2)_q$-C(=$NX^2$)-$NH_2$, where p represents an integer from 1 to 3 and q represents a number from 0 to 2, but also where instead of
>CH-$R^1$ >C=CH-$C_6H_4$-$X^1$ can be present;
X denotes hydrogen, ($C_1$-$C_6$)-alkyl, ($C_1$-$C_6$)-alkoxycarbonyl, ($C_1$-$C_6$)-alkylcarbonyl, ($C_1$-$C_{18}$)-alkylcarbonyloxy-($C_1$-$C_6$)-alkoxycarbonyl, ($C_6$-$C_{14}$)-aryloxycarbonyl, ($C_6$-$C_{14}$)-aryl-($C_1$-$C_6$)-alkoxycarbonyl, cyano, hydroxyl, ($C_1$-$C_6$)-alkoxy, amino or a radical of the formula II

$$R' - NH - \underset{|}{C} = N - R'' \qquad (II)$$

in which R' and R'' independently of one another represent hydrogen, ($C_1$-$C_6$)-alkyl, ($C_1$-$C_6$)-alkoxycarbonyl, ($C_1$-$C_6$)-alkylcarbonyl, ($C_1$-$C_{18}$)-alkylcarbonyloxy-($C_1$-$C_6$)-alkoxycarbonyl, ($C_6$-$C_{14}$)-aryloxycarbonyl, ($C_6$-$C_{14}$)-aryl-($C_1$-$C_6$)-alkoxycarbonyl, cyano, hydroxyl, ($C_1$-$C_6$)-alkoxy or amino;
$X^1$ denotes $-(CH_2)_q$-NH-X or $-(CH_2)_q$-C(=$NX^2$)-$NH_2$;

$X^2$ denotes hydrogen, ($C_1$-$C_6$)-alkyl, ($C_1$-$C_6$)-alkoxycarbonyl, ($C_1$-$C_6$)-alkylcarbonyl, ($C_1$-$C_{18}$)-alkylcarbonyloxy-($C_1$-$C_6$)-alkoxycarbonyl, ($C_6$-$C_{14}$)-aryloxycarbonyl, ($C_6$-$C_{14}$)-aryl-($C_1$-$C_6$)-alkoxycarbonyl, cyano, hydroxyl, ($C_1$-$C_6$)-alkoxy or amino;
$R^2$ denotes hydrogen or ($C_1$-$C_6$)-alkyl;
$R^3$ denotes hydrogen or phenyl;
$R^4$ denotes hydrogen, -$COOR^5$, -CO-N($CH_3$)-$R^5$ or -CO-NH-$R^5$;
$R^5$ denotes hydrogen or ($C_1$-$C_{28}$)-alkyl, which is optionally monosubstituted or polysubstituted by identical or different radicals from the series consisting of hydroxyl, hydroxycarbonyl, aminocarbonyl, mono- or di-($C_1$-$C_{18}$) -alkylaminocarbonyl, amino-($C_2$-$C_{14}$)-alkylaminocarbonyl, amino-($C_1$-$C_3$)-alkylphenyl-($C_1$-$C_3$)-alkylaminocarbonyl, ($C_1$-$C_{18}$)-alkylcarbonylamino-($C_1$-$C_3$)-alkylphenyl-($C_1$-$C_3$)-alkylaminocarbonyl, ($C_1$-$C_{18}$)-alkylcarbonylamino-($C_2$-$C_{14}$)-alkylaminocarbonyl, phenyl-($C_1$-$C_8$)-alkoxycarbonyl, amino, mercapto, ($C_1$-$C_{18}$)-alkoxy, ($C_1$-$C_{18}$)-alkoxycarbonyl, ($C_3$-$C_8$)-cycloalkyl, halogen, nitro, trifluoromethyl or a radical $R^6$;
$R^6$ denotes ($C_6$-$C_{14}$)-aryl, ($C_6$-$C_{14}$)-aryl-($C_1$-$C_8$)-alkyl, a monocyclic or bicyclic 5- to 12-membered heterocyclic ring which can be aromatic, partially hydrogenated or completely hydrogenated and which, as the heteroelement, can contain one, two or three identical or different nitrogen, oxygen or sulphur atoms, or denotes a radical $R^7$, where the aryl radical and, independently thereof, the heterocyclic radical can be optionally monosubstituted or polysubstituted by identical or different radicals from the series consisting of ($C_1$-$C_{18}$)-alkyl, ($C_1$-$C_{18}$)-alkoxy, halogen, nitro and trifluoromethyl;
$R^7$ denotes -$NR^8R^9$, -$OR^8$, -$SR^8$, an amino acid side chain, a natural or unnatural amino acid residue, imino acid residue or dipeptide residue, and also their esters and amides, where free functional groups can be protected by protective groups customary in peptide chemistry, or denotes a radical -$COR^{7'}$, in which $R^{7'}$ is defined as $R^7$;
$R^8$ denotes hydrogen, ($C_2$-$C_{18}$)-alkyl, ($C_6$-$C_{14}$)-aryl-($C_1$-$C_8$)-alkyl, ($C_1$-$C_{18}$)-alkylcarbonyl, ($C_1$-$C_{18}$)-alkoxycarbonyl, ($C_6$-$C_{14}$)-arylcarbonyl, ($C_6$-$C_{14}$)-aryl-($C_1$-$C_8$)-alkylcarbonyl, or ($C_6$-$C_{14}$)-aryl-($C_1$-$C_{18}$)-alkoxycarbonyl, where the alkyl groups can optionally be substituted by an amino group, denotes a natural or unnatural amino acid residue, imino acid residue or a dipeptide residue; and
$R^9$ denotes hydrogen, ($C_1$-$C_{18}$)-alkyl, ($C_6$-$C_{14}$)-aryl or ($C_6$-$C_{14}$)-aryl-($C_1$-$C_8$)-alkyl;

or in which the radicals in the formula I are defined as indicated in Claims 2 to 4, and their physiologically tolerable salts for use as inhibitors of platelet aggregation, metastasis of carcinoma cells or osteoclast binding to the bone surfaces.

**7.** Pharmaceutical preparation, characterised in that it contains one or more of the compounds of the formula I defined in Claim 6 or a physiologically tolerable salt thereof as active compound together with pharmaceutically acceptable excipients and additives and, if appropriate, also one or more other pharmacological active compounds.

**8.** Process for the production of a pharmaceutical preparation containing one or more of the compounds of the formula I defined in Claim 6 or a physiologically tolerable salt thereof, characterised in that these are brought into a suitable administration form together with pharmaceutically acceptable excipients and additives and, if appropriate, also one or more other pharmacological active compounds.

## Revendications

**1.** Composés de formule générale I

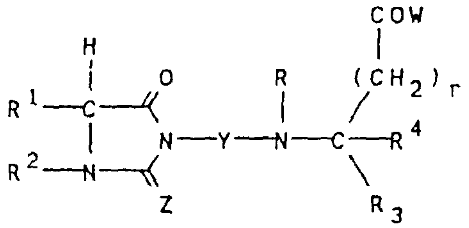

(I)

dans laquelle

Y représente $-(CH_2)_m-CO-$, m représentant un nombre entier allant de 1 à 4, ou

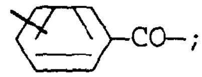

r représente un nombre allant de 0 à 3;

Z représente un atome d'oxygène ou de soufre;

W représente un groupe alcoxy en $C_1$-$C_{28}$, aryl($C_6$-$C_{14}$)-alcoxy($C_1$-$C_8$), aryloxy en $C_6$-$C_{14}$, amino ou mono- ou dialkyl($C_1$-$C_{18}$)amino;

R représente un atome d'hydrogène ou un groupe alkyle en $C_1$-$C_6$;

$R^1$ représente un groupe $-(CH_2)_n-NH-X$, n représentant un nombre entier allant de 1 à 6, $-(CH_2)_p-C_6H_4-(CH_2)_q-NH-X$ ou $-(CH_2)_p-C_6H_4-(CH_2)_q-C(=NX^2)-NH_2$, p étant un nombre entier allant de 1 à 3 et q étant un nombre allant de 0 à 2,
$\rangle C=CH-C_6H_4-X^1$ pouvant cependant être présent au lieu de $\rangle CH-R^1$;

X représente un atome d'hydrogène ou un groupe alkyle en $C_1$-$C_6$, alcoxy($C_1$-$C_6$)-carbonyle, alkyl($C_1$-$C_6$)-carbonyle, alkyl($C_1$-$C_{18}$)-carbonyloxy-alcoxy($C_1$-$C_6$)-carbonyle, aryloxy($C_6$-$C_{14}$)-carbonyle, aryl($C_6$-$C_{14}$)-alcoxy($C_1$-$C_6$)-carbonyle, cyano, hydroxy, alcoxy en $C_1$-$C_6$, amino ou un radical de formule II

$$R'-NH-C(=N-R'')- \qquad (II),$$

dans laquelle R' et R" représentent, indépendamment l'un de l'autre, un atome d'hydrogène ou un groupe alkyle en $C_1$-$C_6$, alcoxy($C_1$-$C_6$)-carbonyle, alkyl($C_1$-$C_6$)-carbonyle, alkyl($C_1$-$C_{18}$)-carbonyloxy-alcoxy ($C_1$-$C_6$)-carbonyle, aryloxy($C_6$-$C_{14}$)-carbonyle, aryl($C_6$-$C_{14}$)-alcoxy($C_1$-$C_6$)-carbonyle, cyano, hydroxy, alcoxy en $C_1$-$C_6$ ou amino;

$X^1$ représente un groupe $-(CH_2)_q-NH-X$ ou $-(CH_2)_q-C(=NX^2)-NH_2$;

$X^2$ représente un atome d'hydrogène ou un groupe alkyle en $C_1$-$C_6$, alcoxy($C_1$-$C_6$)-carbonyle, alkyl($C_1$-$C_6$)-carbonyle, alkyl($C_1$-$C_{18}$)-carbonyloxy-alcoxy($C_1$-$C_6$)-carbonyle, aryloxy($C_6$-$C_{14}$)-carbonyle, aryl($C_6$-$C_{14}$)-alcoxy($C_1$-$C_6$)-carbonyle, cyano, hydroxy, alcoxy en $C_1$-$C_6$ ou amino;

$R^2$ représente un atome d'hydrogène ou un groupe alkyle en $C_1$-$C_6$;

$R^3$ représente un atome d'hydrogène ou le groupe phényle;

$R^4$ représente un atome d'hydrogène ou un groupe $-COOR^5$, $-CO-N(CH_3)-R^5$ ou $-CO-NH-R^5$;

$R^5$ représente un atome d'hydrogène ou un groupe alkyle en $C_1$-$C_{28}$, qui peut éventuellement être une ou plusieurs fois substitué par des radicaux, identiques ou différents, choisis dans l'ensemble constitué par des groupes hydroxy, hydroxycarbonyle, aminocarbonyle, mono- ou dialkyl($C_1$-$C_{18}$) aminocarbonyle, aminoalkyl-($C_2$-$C_{14}$)aminocarbonyle, aminoalkyl($C_1$-$C_3$)-phénylalkyl($C_1$-$C_3$)aminocarbonyle, alkyl($C_1$-$C_{18}$)-carbonylaminoalkyl($C_1$-$C_3$)-phényl-alkyl($C_1$-$C_3$)aminocarbonyle, alkyl ($C_1$-$C_{18}$)-carbonylamino-alkyl($C_2$-$C_{14}$)aminocarbonyle, phényl-alcoxy($C_1$-$C_8$)-carbonyle, amino, mercapto, alcoxy en $C_1$-$C_{18}$, alcoxy($C_1$-$C_{18}$)-carbonyle, cycloalkyle en $C_3$-$C_8$, des atomes d'halogène, les groupes nitro, trifluorométhyle ou un radical $R^6$;

$R^6$ représente un groupe aryle en $C_6$-$C_{14}$, aryl($C_6$-$C_{14}$)-alkyle($C_1$-$C_8$), un cycle hétérocyclique mono- ou bicyclique à 5-12 chaînons, qui peut être aromatique, partiellement hydrogéné ou totalement hydrogéné, et qui peut contenir comme hétéroatome 1, 2 ou 3 atomes d'azote, d'oxygène ou de soufre, identiques ou différents, ou représente un radical $R^7$, le radical aryle et, indépendamment de celui-ci, le radical hétérocyclique, pouvant éventuellement être une ou plusieurs fois substitués par des substituants, identiques ou différents, choisis parmi les atomes d'halogène et des groupes alkyle en $C_1$-$C_{18}$, alcoxy en $C_1$-$C_{18}$, nitro et trifluorométhyle;

$R^7$ représente un groupe $-NR^8R^9$, $-OR^8$, $-SR^8$, une chaîne latérale d'aminoacide, un reste d'aminoacide, d'imino-acide ou de dipeptide, naturel ou non naturel, ainsi que leurs esters et amides, des groupes fonctionnels libres pouvant être protégés par des groupes protecteurs usuels dans la chimie des peptides, ou représente un radical $-COR^{7'}$, dans lequel $R^{7'}$ est défini comme $R^7$;

$R^8$ représente un atome d'hydrogène ou un groupe alkyle en $C_2$-$C_{18}$, aryl($C_6$-$C_{14}$)-alkyle($C_1$-$C_8$), alkyl ($C_1$-$C_{18}$)-carbonyle, alcoxy($C_1$-$C_{18}$)-carbonyle, aryl($C_6$-$C_{14}$)-carbonyle, aryl($C_6$-$C_{14}$)-alkyl($C_1$-

$C_8$)-carbonyle, aryl-($C_6$-$C_{14}$)-alcoxy($C_1$-$C_{18}$)-carbonyle, les groupes alkyle pouvant éventuellement être substitués par un groupe amino, ou représente un reste d'aminoacide, d'imino-acide ou de dipeptide naturel ou non naturel; et

$R^9$ représente un atome d'hydrogène ou un groupe alkyle en $C_1$-$C_{18}$, aryle en $C_6$-$C_{14}$ ou aryl($C_6$-$C_{14}$)-alkyle($C_1$-$C_8$) ; ainsi que leurs sels physiologiquement acceptables, à l'exclusion des composés de formule générale I dans lesquels simultanément

Y représente $-CH_2-CO-$;

r représente le nombre 1;

Z représente un atome d'oxygène;

W représente le groupe tert-butoxy;

R, $R^2$ et $R^3$ représentent des atomes d'hydrogène;

$R^1$ représente $-(CH_2)_k-NHX^a$, k représentant 3 ou 4,

$X^a$ représentant un atome d'hydrogène ou un groupe alkyle en $C_1$-$C_6$ ou un radical de formule

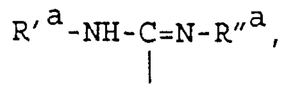

dans laquelle $R'^a$ et $R''^a$ représentent, indépendamment l'un de l'autre, un atome d'hydrogène ou un groupe alkyle en $C_1$-$C_6$;

$R^4$ représente $-CONHR^5$.

**2.** Composés selon la revendication 1, caractérisés en ce que, dans la formule générale I,

Y représente $-(CH_2)_m-CO-$, m représentant 1 ou 2, ou

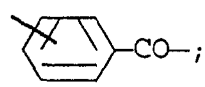

r est égal à 1;

Z représente un atome d'oxygène ou de soufre;

W représente un groupe alcoxy en $C_1$-$C_4$, en particulier méthoxy, éthoxy ou 2-propyloxy;

R représente un atome d'hydrogène;

$R^1$ représente $-(CH_2)_n-NH-X$, n représentant un nombre entier allant de 1 à 4, $-CH_2-C_6H_4-(CH_2)_q-NH-X$ ou $-CH_2-C_6H_4-(CH_2)_q-C-(=NX^2)-NH_2$, q étant 0 ou 1, $>C=CH-C_6H_4-X^1$ pouvant cependant être présent au lieu de $>CH-R^1$;

X représente un atome d'hydrogène ou un groupe alcoxy($C_1$-$C_6$)-carbonyle, alkyl($C_1$-$C_6$)-carbonyle, aryl($C_6$-$C_{14}$)-alcoxy($C_1$-$C_6$)-carbonyle ou un radical de formule II

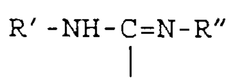 (II),

dans laquelle R' et R" représentent, indépendamment l'un de l'autre, un atome d'hydrogène ou un groupe alcoxy($C_1$-$C_6$)-carbonyle, alkyl($C_1$-$C_6$)-carbonyle ou aryl($C_6$-$C_{14}$)-alcoxy($C_1$-$C_6$)carbonyle;

$X^1$ représente $-(CH_2)_q-NH-X$ ou $-C(=NX^2)-NH_2$, q représentant 0 ou 1;

$X^2$ représente un atome d'hydrogène ou un groupe alcoxy($C_1$-$C_6$)-carbonyle, alky($C_1$-$C_6$)-carbonyle ou aryl($C_6$-$C_{14}$)-alcoxy($C_1$-$C_6$)-carbonyle;

$R^2$ représente un atome d'hydrogène ou le groupe méthyle;

$R^3$ représente un atome d'hydrogène; et

$R^4$ représente $-CO-NH-R^5$, $-NH-R^5$ représentant un reste d'α-aminoacide, un ω-amino-alkyl($C_2$-$C_8$)amide de celui-ci ou un ester alkylique en $C_1$-$C_8$ ou benzylique de celui-ci.

**3.** Composés selon la revendication 2, caractérisés en ce que les restes d'α-aminoacides représentés par $-NH-R^5$

sont les restes de la valine, de la lysine, de la phénylalanine, de la phénylglycine ou de la 4-chlorophénylglycine.

**4.** Composés selon la revendication 2, caractérisés en ce que l'ω-amino-alkyl($C_2$-$C_8$)amide est le 4-aminobutylamide.

**5.** Procédé pour la préparation des composés des revendications 1 à 4, caractérisé en ce que l'on effectue une condensation de fragments d'un composé de formule générale III

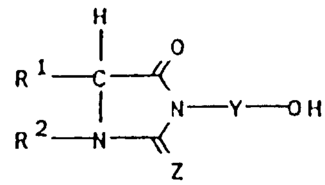

(III)

avec un composé de formule générale IV

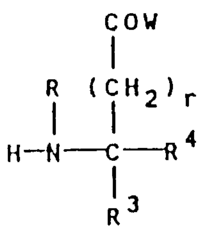

(IV)

les radicaux R, $R^1$ à $R^4$ et r, Y, Z et W étant définis comme indiqué dans la revendication 1.

**6.** Composés de formule générale I

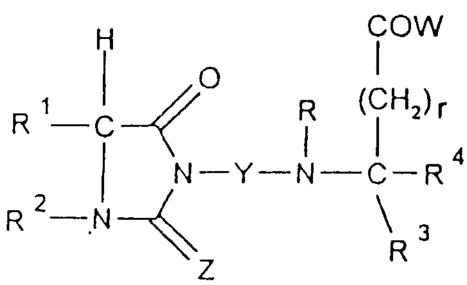

(I)

dans laquelle

Y représente $-(CH_2)_m$-CO-, m représentant un nombre entier allant de 1 à 4, ou

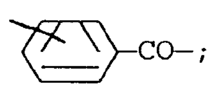

r représente un nombre allant de 0 à 3;
Z représente un atome d'oxygène ou de soufre;
W représente un groupe alcoxy en $C_1$-$C_{28}$, aryl($C_6$-$C_{14}$)-alcoxy($C_1$-$C_8$), aryloxy en $C_6$-$C_{14}$, amino ou mono- ou dialkyl($C_1$-$C_{18}$)amino;
R représente un atome d'hydrogène ou un groupe alkyle en $C_1$-$C_6$;
$R^1$ représente un groupe $-(CH_2)_n$-NH-X, n représentant un nombre entier allant de 1 à 6, $-(CH_2)_p-C_6H_4-(CH_2)_q$-NH-X ou $-(CH_2)_p-C_6H_4-(CH_2)_q-C(=NX^2)-NH_2$, p étant un nombre entier allant de 1 à 3 et q étant un nombre allant de 0 à 2,

$>C=CH-C_6H_4-X^1$ pouvant cependant être présent au lieu de $>CH-R^1$;

X représente un atome d'hydrogène ou un groupe alkyle en $C_1$-$C_6$, alcoxy($C_1$-$C_6$)-carbonyle, alkyl($C_1$-$C_6$)-carbonyle, alkyl($C_1$-$C_{18}$)-carbonyloxy-alcoxy($C_1$-$C_6$)-carbonyle, aryloxy($C_6$-$C_{14}$)-carbonyle, aryl($C_6$-$C_{14}$)-alcoxy ($C_1$-$C_6$)-carbonyle, cyano, hydroxy, alcoxy en $C_1$-$C_6$, amino ou un radical de formule II

$$R'-NH-\underset{|}{C}=N-R'' \qquad (II),$$

dans laquelle R' et R" représentent, indépendamment l'un de l'autre, un atome d'hydrogène ou un groupe alkyle en $C_1$-$C_6$, alcoxy($C_1$-$C_6$)-carbonyle, alkyl($C_1$-$C_6$)-carbonyle, alkyl($C_1$-$C_{18}$)-carbonyloxy-alcoxy($C_1$-$C_6$)-carbonyle, aryloxy($C_6$-$C_{14}$)-carbonyle, aryl($C_6$-$C_{14}$)-alcoxy($C_1$-$C_6$)-carbonyle, cyano, hydroxy, alcoxy en $C_1$-$C_6$ ou amino;

$X^1$ représente un groupe $-(CH_2)_q-NH-X$ ou $-(CH_2)_q-C(=NX^2)-NH_2$;

$X^2$ représente un atome d'hydrogène ou un groupe alkyle en $C_1$-$C_6$, alcoxy($C_1$-$C_6$)-carbonyle, alkyl($C_1$-$C_6$)-carbonyle, alkyl($C_1$-$C_{18}$)-carbonyloxy-alcoxy($C_1$-$C_6$)-carbonyle, aryloxy($C_6$-$C_{14}$)-carbonyle, aryl($C_6$-$C_{14}$)-alcoxy ($C_1$-$C_6$)-carbonyle, cyano, hydroxy, alcoxy en $C_1$-$C_6$ ou amino;

$R^2$ représente un atome d'hydrogène ou un groupe alkyle en $C_1$-$C_6$;

$R^3$ représente un atome d'hydrogène ou le groupe phényle; $R^4$ représente un atome d'hydrogène ou un groupe $-COOR^5$, $-CO-N(CH_3)-R^5$ ou $-CO-NH-R^5$;

$R^5$ représente un atome d'hydrogène ou un groupe alkyle en $C_1$-$C_{28}$, qui peut éventuellement être une ou plusieurs fois substitué par des radicaux, identiques ou différents, choisis dans l'ensemble constitué par des groupes hydroxy, hydroxycarbonyle, aminocarbonyle, mono-ou dialkyl($C_1$-$C_{18}$)aminocarbonyle, aminoalkyl ($C_2$-$C_{14}$)aminocarbonyle, aminoalkyl ($C_1$-$C_3$)-phénylalkyl($C_1$-$C_3$)aminocarbonyle, alkyl($C_1$-$C_{18}$)-carbonylaminoalkyl($C_1$-$C_3$)-phényl-alkyl($C_1$-$C_3$)aminocarbonyle, alkyl($C_1$-$C_{18}$)-carbonylamino-alkyl($C_2$-$C_{14}$)aminocarbonyle, phényl-alcoxy($C_1$-$C_8$)-carbonyle, amino, mercapto, alcoxy en $C_1$-$C_{18}$, alcoxy($C_1$-$C_{18}$)-carbonyle, cycloalkyle en $C_3$-$C_8$, des atomes d'halogène, les groupes nitro, trifluorométhyle ou un radical $R^6$;

$R^6$ représente un groupe aryle en $C_6$-$C_{14}$, aryl($C_6$-$C_{14}$)-alkyle($C_1$-$C_8$), un cycle hétérocyclique mono- ou bicyclique à 5-12 chaînons, qui peut être aromatique, partiellement hydrogéné ou totalement hydrogéné, et qui peut contenir comme hétéroatome 1, 2 ou 3 atomes d'azote, d'oxygène ou de soufre, identiques ou différents, ou représente un radical $R^7$, le radical aryle et, indépendamment de celui-ci, le radical hétérocyclique, pouvant éventuellement être une ou plusieurs fois substitués par des substituants, identiques ou différents, choisis parmi les atomes d'halogène et des groupes alkyle en $C_1$-$C_{18}$, alcoxy en $C_1$-$C_{18}$, nitro et trifluorométhyle;

$R^7$ représente un groupe $-NR^8R^9$, $-OR^8$, $-SR^8$, une chaîne latérale d'aminoacide, un reste d'aminoacide, d'iminoacide ou de dipeptide, naturel ou non naturel, ainsi que leurs esters et amides, des groupes fonctionnels libres pouvant être protégés par des groupes protecteurs usuels dans la chimie des peptides, ou représente un radical $-COR^{7'}$, dans lequel $R^{7'}$ est défini comme $R^7$;

$R^8$ représente un atome d'hydrogène ou un groupe alkyle en $C_2$-$C_{18}$, aryl($C_6$-$C_{14}$)-alkyle($C_1$-$C_8$), alkyl($C_1$-$C_{18}$) carbonyle, alcoxy($C_1$-$C_{18}$) -carbonyle, aryl($C_6$-$C_{14}$)carbonyle, aryl($C_6$-$C_{14}$)-alkyl($C_1$-$C_8$)-carbonyle, aryl($C_6$-$C_{14}$)-alcoxy($C_1$-$C_{18}$)-carbonyle, les groupes alkyle pouvant éventuellement être substitués par un groupe amino, ou représente un reste d'aminoacide, d'iminoacide ou de dipeptide naturel ou non naturel; et

$R^9$ représente un atome d'hydrogène ou un groupe alkyle en $C_1$-$C_{18}$, aryle en $C_6$-$C_{14}$ ou aryl($C_6$-$C_{14}$)-alkyle($C_1$-$C_8$) ; ou dans lesquels les radicaux dans la formule générale I sont définis comme indiqué dans les revendication 2 à 4, ainsi que leurs sels physiologiquement acceptables, pour utilisation en tant qu'inhibiteurs de l'agrégation des thrombocytes, de la formation de métastases de cellules de carcinomes ou de la fixation d'ostéoclastes à la surface des os.

**7.** Composition pharmaceutique, caractérisée en ce qu'elle contient, en tant que substance active, un ou plusieurs des composés de formule générale I, définis dans la revendication 6, ou un sel physiologiquement acceptable de ceux-ci, conjointement avec des additifs et véhicules pharmaceutiquement acceptables et éventuellement encore une ou plusieurs autres substances pharmacologiquement actives.

**8.** Procédé pour la préparation d'une composition pharmaceutique, contenant un ou plusieurs des composés de formule générale I définis dans la revendication 6 ou un sel physiologiquement acceptable de ceux-ci, caractérisé en ce qu'on les met sous une forme d'administration appropriée, conjointement avec des additifs et véhicules pharmaceutiquement acceptables et éventuellement encore une ou plusieurs autres substances pharmacologiquement actives.